# NEW EUROPEAN PATENT SPECIFICATION

(11) **EP 0 301 670 B2**
(45) Date of publication and mention of the opposition decision: **14.06.2000**
(45) Mention of the grant of the patent: 18.11.1993
(21) Application number: 88201632.2
(22) Date of filing: 28.07.1988
(51) Int. Cl.: C12N 15/62, C12N 15/81, C12N 9/24, C12N 9/64, C12N 9/72, C12P 21/00, C12N 1/16, C12N 5/00

(54) **Kluyveromyces as a host strain**
Kluyveromyces als Wirtsstamm
Kluyveromyces comme souche hôte

(30) Priority: 28.07.1987 US 78539
(43) Date of publication of application: 01.02.1989
(73) Proprietor: GIST-BROCADES N.V., NL-2611 XT Delft (NL)
(72) Inventor: van den Berg, Johannes A., Dr., NL-2811 AD Reeuwijk (NL); van Ooyen, Albert J.J., Dr., NL-2271 AR Voorburg (NL); Rietveld, Krijn, Dr., NL-3132 TL Vlaardingen (NL)
(74) Representative: Jaenichen, Hans-Rainer, Dr.

(56) References cited:
- EP-A- 0 077 109
- EP-A- 0 096 430
- EP-A- 0 096 910
- EP-A- 0 116 201
- EP-A- 0 241 435
- EP-B- 0 088 632
- EP-B- 0 095 986
- US-A- 4 657 857
- Concise Encyclopedia of Biochemistry, de Gruyter Ed., 1983, p. 426
- Science, Vol. 229, 1985, PP. 1219-1224; R.A. Smith et al
- Current Genetics, vol. 6, 1982, pp. 123-128; S. Das and C. Hollenberg

## Description

### Technical Field

This invention relates to methods for preparing and using Kluyveromyces for the production of polypeptides of interest which are secreted into the growth medium. The invention is exemplified by sequences useful in the production of chymosin and precursors thereof, tissue plasminogen activator (t-Pa), and human serum albumin (HSA), in Kluyveromyces.

### Background of the Invention

The bright promise of production of peptides in microorganisms has been tarnished by a number of factors. In many instances, where the peptide has been produced and retained in the cytoplasm, inclusion bodies have resulted requiring denaturation and renaturation of the protein, frequently with only partial or little success. In other instances, the peptide has been subjected to substantial degradation, so that not only are yields low, but also complicated mixtures are obtained which are difficult to separate. As a potential solution to these difficulties, the possibility of secretion of the desired peptide into the nutrient medium has been investigated. Secretion has met with limited success, since not all proteins have been found to be capable of secretion in the host which have been employed. Even when secreted, the processing of the peptide may result in a product which differs from the composition and/or conformation of the desired peptide. There is, therefore, substantial interest in being able to develop systems for the efficient and economic production of active peptides under conditions which allow for the use of the peptides in a wide variety of environments, both in vitro and in vivo.

### RELEVANT LITERATURE

European Patent Application (EPA) 0096430 discloses Kluyveromyces as a host for cloning and expression of foreign genes. However, no mention was made of the capability of secreting proteins into the growth medium.

The leader sequence of amyloglucosidase for Aspergillus is described by Boyle et al., EMBO J. (1984) 3:1581-1585 and Innis et al., Science (1985) 228:21-26. Lactase promoters are described by Breunig et al., Nucleic Acids Res. (1984) 12:2327-2341. The use of signal peptides associated with mating-type α-factor and of the enzymes invertase and acid phosphatase to direct the secretion of heterologous proteins in Saccharomyces has been described in EP-A-0123544 and by Smith et al., Science (1985) 229:1219.

Production of preprochymosin, prochymosin and chymosin in Saccharomyces has been studied by Mellor et al., Gene (1983) 24:1-14. When prochymosin is made intracellularly in Saccharomyces, only a few percentage of the prochymosin obtained is activatable. See Moir et al. in Developments in Industrial Biology (1985) 26:75-85; Mellor et al., Gene (1983) 24:1-14; Kingsman et al. in Biotechnology and Genetic Engineering Reveiws Vol. 3 (1985) 376-416. The aggregated prochymosin produced by Saccharomyces required complicated methods of denaturation and renaturation to solubilize the prochymosin. See WO 83/04418 and EP-A-0114506.

### SUMMARY OF THE INVENTION

The present invention addresses the need for improving the production and recovery of a protein of interest by providing the embodiments characterized in the claims. Thus, in accordance with the present invention, peptide production systems are provided comprising Kluyveromyces host strains, expression casssemes which include efficient transcriptional initiation and termination regions for use in Kluyveromyces and a gene, optionally containing a signal sequence for secretion, under the transcriptional and translational regulation of the regulatory regions. The cassettes are introduced into the Kluyveromyces host strain under conditions whereby the resulting transformants stably maintain the expression cassettes. Naturally occurring DNA and synthetic genes may be employed for the production of peptides of interest.

### BRIEF DESCRIPTION OF THE FIGURES

Figure 1 is a diagram of the plasmid pGBTe418;
Figure 2 is a diagram of the plasmic pGB901;
Figure 3 is a description of the synthesized oligonucleotides for the signal sequence adapted from the amyloglucosidase signal sequence;
Figure 4 is a description of a synthetic signal sequence;
Figure 5 is an immunoblot showing the secretion of prochymosin by a lactis;
Figure 6 is the sequence of the entire BamHI insert from pDM100PC comprising the fusion peptide of the α-factor of S. cerevisiae and prochymosin and transcriptional regulatory regions;
Figure 7 is a restriction map of plasmid pKS105;
Figure 8 shows the strategy used to design oligonucleotide process used to identify K. lactis α-factor DNA.
Figure 9 is the complete sequence of a DNA fragment encoding the K. lactis α-factor;
Figure 10 is a description of plasmids employed for expression of the fusion of the α-factor signal sequence and the prochymosin structual gene;
Figure 11 shows the sequences around the junctions in α-factor/prochymosin fusions;
Figure 12 is the sequence of the BamHI/SaII insert of pAB309.
Figure 13 represents the sequences of the primers for mutagenesis of K. lactis α-factor leader DNA;
Figure 14 is a diagram of the plasmid pUCG418;
Figure 15 is a diagram of the plasmid pGBtPA1;
Figure 16 shows the secretion of human t-PA by K. lactis as analysed on a SDS-polyacrylamide gel overlayered with a plasminogen/fibrin-agarose gel;
Figure 17 is a diagram of the plasmid pGBtPA2;
Figure 18 is a diagram of the plasmid pGBHSA3;
Figure 19 shows the secretion of HSA by K. lactis as analysed on a 10% polyacrylamide gel.

### DESCRIPTION OF THE SPECIFIC EMBODIMENTS

In accordance with the subject invention, a method is provided which allows for the efficient and economic production of polypeptides by Kluyveromyces yeast cells. The expression cassettes employed in accordance with the method of the invention have transcriptional and translational regulatory sequences functional in a Kluyveromyces host cell and an open reading frame coding for a peptide of interest under the transcriptional and translational control of the regulatory regions. The open reading frame also may include a leader sequence recognized by the Kluyveromyces host which provides for secretion of the polypeptide into the growth medium. The Kluyveromyces cells used may be either laboratory or industrial strains.

The expression cassette will include in the the 5'-3' direction of transcription, a transcriptional and translational initiation regulatory region, and open reading frame encoding a peptide of interest, desirably having a signal sequence for secretion recognized by Kluyveromyces, and a translational termination region. The expression cassette will further comprise a transcriptional termination regulatory region. The initation and termination regulatory regions are functional in Kluyveromyces and provides for efficient expression of the peptide of interest without undesirable effects on the viability and proliferation of the Kluyveromyces host.

The transcriptional and translational initiation regulatory region may be homologous or heterologous to Kluyveromyces. Of particular interest are transcriptional initiation regions from genes which are present in Kluyveromyces or other yeast species, such as Saccharomyces, for example, cerevisiae, Schizosaccharomyces, Candida, etc., or other fungi, for example, filamentous fungi such as Aspergillus, Neurospora, Penicillium, etc. The transcriptional intiation regulatory regions may be obtained for example from genes in the glycolytic pathway, such as alcohol dehydrogenase, glyceraldehyde-3-phosphate dehydrogenase phosphoglucoisomerase, phosphoglycerate kinase, etc., or regulatable genes such as acid phosphate, lactase, glucoamylase, etc.

Any one of a number of regulatory sequences may be preferred in a particular situation, depending upon whether constitutive or induced transcription is desired, the particular efficiency of the promoter in conjunction with the open reading frame of interest, the ability to join a strong promoter with a control region from a different promoter which allows for inducible transcription, ease of construction, and the like. These regulatory regions find ample precedent in the literature. See, for example, EP-A-0164566, incorporated herein by reference.

Secretion of heterologous proteins in genetically modified microorganisms is generally accomplished by one of two methods. In the first, the leader sequence is homologous to the protein in the second, the leader sequence is homologous to the host organism. Other alternatives of particular interest in the present invention for secretion of heterologous proteins in Kluyveromyces include the use of a leader sequence heterologous to both Kluyveromyces and the peptide of interest, or a synthetic leader sequence specifically designed. The DNA encoding the former leader sequence can be obtained by isolation or made synthetically. Thus, the open reading frame usually will include a wild-type or mutated gene, where the signal sequence is normally associated with remainder of the coding sequence, a hybrid or chimeric open reading frame, where the signal sequence is normally not associated with the remaining portion of the open reading frame, or a synthetic sequence, where the signal sequence and the remainder of the open reading frame are synthesized to provide for preferred codons. convenient restriction sites, novel amino acid sequences, and the like, or combinations thereof. Signal sequences which may be employed may be obtained from genes, such as α-factor invertase, amyloglucosidase, native or wild type signal sequences present in structural genes and recognized by Kluyveromyces, Saccharomyces, other fungi, e.g. Neurospora, Aspergillus, and other eukaryotes.

Of particular interest is the use of a signal sequence which provides for secretion of the peptide of interest into the nutrient medium, rather than into periplasmic space. For the most part, the signal sequence will be the 5'-terminus of the open reading frame. However, in some situations, it may be desirable to have the signal sequence internal to the open reading frame. For use of internal signal sequences for secretion, see U.S. Patent No. 4,338,397 and Perara and Lingappa, J. Cell Biology (1985) 101:2292-2301. Genes into which the open reading frame of interest may be inserted include highly expressed constitutive genes, for example, genes encoding enzymes of the glycolytic pathway, or highly expressed regulatable genes such as lactase. amyloglucosidase, or the like.

For optimal gene expression, the nucleotide sequences surrounding the translational initiation codon ATG have been found to be important in yeast cells and in animal cells. For example, M. Kozak, Microbiol. Revs. (1983) 47:1-45 has studied extensively the effect of these regions on the expression of insulin in COS cells. Similary, specific nucleotides are found more frequently in highly expressed yeast proteins than others indicating the important effect of these nucleotides on the level of expression of these genes.

For optimal gene expression of exogenous genes it will be important to modify the nucleotide sequences surrounding the initiation codon ATG. This can be done by sitedirected mutagenesis or by fusing the exogenous gene in frame to an endogenous Kluyveromyces gene, preferably a highly expressed gene, such as the lactase gene.

Normally, it will be desirable to provide that the signal leader is cleaved from the peptide of interest during the secretory process, rather than subsequent to the secretory process, although either procedure may find use. Usually, the processing signal employed will be the processing signal naturally occurring with the signal sequence or a processing signal which has been modified from the naturally occurring one, which is still effective for providing for a peptide signal resulting in hydrolytic cleavage of the signal peptide and processing signal peptide from the peptide of interest. Various processing signals have been sequenced and defined, such as α-factor (see for example U.S. Patent No. 4,546,082, which is incorporated herein by reference). amyloglucosidase. α-amylase. etc. In some instances, other peptidase-recognized sequences may be employed which may required subsequent cleavage for isolation of the desired peptide. These sequences include dibasic peptides. e.g. KR, (D)₄K, and EA, which are cleaved by KEX2, bovine enterokinase, and a yeast membrane peptidase, respectively.

The peptide of interest may be native to the host or heterologous, being derived from prokaryotic or eukaryotic sources, which eukaryotic sources may involve fungi, protists. vertebrates, non-vertebrates. and the like. The peptide products may include enzymes, such as lactase. α-amylase, β-amylase. amyloglucosidase, chymosin, etc., mammalian peptides, such as hormones, interleukins, cytokines. cachexin, growth factors, e.g. platelet derived, epidermal, skeletal, etc., growth hormone, follicle stimulating hormone, interferons (α-, β-, and γ-), blood factors such as factor V, VI, VII, VIII (vW or c), IX, X, XI or XII, plasminogen activator (tissue or urinary), serum albumin, e.g. human serum albumin, colony growth factor (e.g. GM), erythropoietin, thaumatin, insulin, etc.

These structural genes may be obtained in a variety of ways. Where the amino acid sequence is known, the structural gene may be synthesized in whole or in part, particularly where it is desirable to provide yeast-preferred codons. Thus, all or a portion of the open reading frame may be synthesized using codons preferred by Kluyveromyces. Preferred codons may be determined by those codons which are found in the proteins produced in greatest amount by the Kluyveromyces host e.g. glycolytic enzymes. Methods for synthesizing sequences and bringing the sequences together are well established in the literature. Where a portion of the open reading frame is synthesized, and a portion is derived from natural sources, the synthesized portion may serve as a bridge between two naturally occurring portions, or may provide a 3'-terminus or a 5'-terminus. Particularly where the signal sequence and the open reading frame encoding the peptide are derived from different genes, synthetic adaptors commonly will be employed. In other instances, linkers may be employed, where the various fragments may be inserted at different restriction sites or substituted for a sequence in the linker.

For the most part, some or all of the open reading frame will be from a natural source. Methods for identifying sequences of interest have found extensive exemplification in the literature, although in individual situations, different degrees of difficulty may be encountered. Various techniques involve the use of probes, where at least a portion of the naturally occurring amino acid sequence is known, where genomic or cDNA libraries may be searched for complementary sequences. Alternatively, differential transcription can be detected when the gene of interest can be induced or when cells are from the same host but of different differentiation, by comparing the messenger RNA's produced. Other techniques have also been exemplified.

The termination region may be derived from the 3'-region of the gene from which the initiation region was obtained or from a different gene. A large number of termination regions are known and have been found to be satisfactory in a variety of hosts from the same and different genera and species. The termination region is usually selected more as a matter of convenience rather than because of any particular property. Preferably, the termination region will be derived from a yeast gene, particularly Saccharomyces or Kluyveromyces.

In developing the expression cassette, the various fragments comprising the regulatory regions and open reading frame may be subjected to different processing conditions, such as ligation, restriction, resection, in vitro mutagenesis, primer repair, use of linkers and adaptors, and the like. Thus, nucleotide transitions, transversions, insertions, deletions, or the like, may be performed on the DNA which is employed in the regulatory regions and/or open reading frame.

During the construction of the expression cassette, the various fragments of the DNA will usually be cloned in an appropriate cloning vector, which allows for expansion of the DNA, modification of the DNA or manipulation by joining or removing of the sequences, linkers, or the like. Normally, the vectors will be capable of replication in at least a relatively high copy number in E. coli. A number of vectors are readily available for cloning, including such vectors as pBR322, pACYC184, pUC7-19, M13, Charon 4A, and the like.

The cloning vectors are characterized by having an efficient replication system functional in E. coli. Also, the cloning vector will have at least one unique restriction site, usually a plurality of unique restriction sites and may also include multiple restriction sites, particularly two of the same restriction sites for substitution. In addition, the cloning vector will have one or more markers which provide for selection for transformants. The markers will normally provide for resistance to cytotoxic agents such as antibiotics, heavy metals, toxins or the like, complementation of an auxotrophic host, or immunity to a phage. By appropriate restriction of the vector and cassette, and, as appropriate, modification of the ends, by chewing back or filling in overhangs, to provide for blunt ends, by addition of linkers, by tailing, complementary ends can be provided for ligation and joining of the vector to the expression cassette or component thereof.

After each manipulation of the DNA in the development of the cassette, the plasmid will be cloned and isolated and, as required, the particular cassette component analyzed as to its sequence to ensure that the proper sequence has been obtained. Depending upon the nature of the manipulation, the desired sequence may be excised from the plasmid and introduced into a different vector or the plasmid may be restricted and the expression cassette component manipulated, as appropriate.

In some instances a shuttle vector will be employed where the vector is capable of replication in different hosts requiring different replication systems. This may or may not require additional markers which are functional in the two hosts. Where such markers are required, these can be included in the vector, where the plasmid containing the cassette, the two replication systems, and the marker(s) may be transferred from one host to another, as required. In the present situation, the second replication system would be a replication system functional in Kluyverornyces. The replication systems which may be used may be derived from plasmids, e.g. pKD1 from Kluyveromyces drosophilarum, viruses, or the chromosome of Kluyveromyces or other species, particularly one associated with Kluyveromyces, such as Saccharomyces. Thus, replication systems include the replication system of the 2 micron plasmid found in Saccharomyces and an autonomously replicating sequence (ARS) gene, for exarnple when used in conjunction with a centromere sequence, or the like. If desired, regions of homology may be provided to encourage integration of the expression cassette into the genome of the Kluyveromyces host.

Of particular interest in the constructs of the subject invention is a sequence derived from Kluyveromyces DNA chromosomes referred to as KARS, which provide for high transformation frequency. The KARS gene may be obtained by screening a library of Kluyveromyces DNA fragments for enhanced transformation efficiency. In this manner, fragments can be obtained which contain KARS sequences, which fragments can be further modified by restriction, resection, or primer repair, to provide a fragment of approximately 200 bp and not more than about 5000 bp, more usually from about 200 bp to 2000 bp which provides for enhanced transformation efficiency. The presence of the KARS gene can provide transformation of K. lactis auxotrophic species to prototophy at a frequency of at least about 10³ per microgram of DNA, usually at a frequency of 10⁴ per microgram of DNA or higher.

The manner of transformation of E. coli with the various DNA constructs (plasmids and viruses) for cloning is not critical to this invention. Conjugation, transduction, transfection or transformation, e.g. calcium chloride or phosphate mediated transformation, may be employed. By contrast, for yeast, for the most part the prior art has relied on transformation of protoplasts employing combinations of calcium ions and polyethylene glycol of from about 2000 to 8000, usually 4000 to 7000 daltons.

An alternative method of transformation involves growing Kluyveromyces in a standard yeast nutrient medium to a density of 1 to 25, desirably 4 to 10 OD₆₁₀. The Kluyveromyces cells are then harvested, washed and pretreated with chaotropic ions; particularly the alkali metal ions lithium, cesium or rubidium, particularly as the chloride or sulfate, more particularly the lithium salts, at concentrations of about 2 mM to 1M, preferably about 0.1 M. After incubating the cells with the chaotropic ion(s), the DNA is added and the incubation is prolonged for a short period of time at a moderate temperature, generally from about 20°C to 35°C, for about 5 to 60 min. Then polyethylene glycol is added, desirably at a concentration to about 25 to 50%, where the entire medium may be diluted by adding an equal volume of a polyethylene glycol concentrate to result in the desired final concentration. The polyethylene glycol will be of from about 2000 to 8000 daltons, preferably about 4000 to 7000 daltons. Incubation will generally be for a relatively short time, generally from about 5 to 60 min. Desirably, the incubation medium is subjected to a heat treatment of from about 1 to 10 min, at about 35°C to 45°C, preferably about 42°C.

For selection, any useful marker may be used, although the number of markers useful with Kluyveromyces is narrower than the markers used for Saccharomyces. Desirably, resistance to kanamycin and the aminoglycoside G418 are of interest, as well as complementation of a gene in the tryptophan metabolic pathway, particularly TRP1 or in the lactose metabolic pathway, particularly LAC4.

Although a marker for selection is highly desirable for convenience, other procedures for screening transformed cells have been described. See for example G. Reipen et al., Current Genetics (1982) 5:189-193. Besides the use of an indicator enzyme such as β-lactamase, transformed cells may be screened by the specific products they make. In the case of chymosin, for example, synthesis of the product may be determined by an immunological or an enzymatic method.

The vector used may be capable of extrachromosomal maintenance in Kluyveromyces or result in integration into the Kluyveromyces gene. It has been found that the 2 micron plasmid replication system from Saccharomyces provides for extrachromosomal maintenance in Kluyveromyces. In addition, one may use a combination of a centromere, such as the Saccharomyces CEN3 and a high transformation frequency sequence, such as ARS or KARS. If selective maintenance is provided, such as complementation or resistance to an antibiotic to which Kluyveromyces is susceptible, the ARS-like sequences will usually suffice for extrachromosomal maintenance.

For large scale fermentation even a small loss of plasmid stability will greatly affect the final yield of the desired protein. To increase the stability of recombinant molecules in host cells, for example Kluyveromyces, integration of the recombinant molecules into the host chromosome may be used.

Where integration is desired, it will usually be desirable to have a sequence homologous to a sequence of the chromosome of the host, so that homologous recombination may occur. It is understood, that random integration also occurs, so that the homologous sequence is optional. Where an homologous sequence is employed, the homologous sequence will usually be at least about 200 bp and may be 1000 bp or more. In addition, where integration is involved, one may wish to have amplification of the structural gene. Amplification has been achieved by providing for a gene in tandem with the desired structural gene, which provide for selective advantage for the host in a selective medium. Thus, the genes expressing dihydrofolate reductase, metallothioneins, thymidine kinase, etc., have proven useful in a variety of hosts to provide for amplification, where the gene provides protection from a toxin, such as methotrexate, heavy metals, such as copper and mercury, and the like.

Vectors of interest providing for stable replication include KARS vectors originating from K. lactis, e.g. pKARS12 and pKARS2, which plasmids comprise a K. lactis DNA fragment containing the KARS 12 or KARS2 sequence in the S. cerevisiae plasmid YRp7. A vector employed for integration is, for example. pL4, a hybrid plasmid of the ARS1 carrying plasmid YRp7 and K. lactis Xhol DNA fragment carrying the LAC4 gene. See EP-A 0096430.

Plasmids of particular interest include plasmids having the 2 micron plasmid replication system, the LAC4 gene, the Tn601 and Tn5 kanamycin resistance gene, which also provides resistance to the antibiotic G418 in Kluyveromyces (Jimenez and Davis, Nature (1980) 287:869-871). This plasmid provides for autonomous replication in Kluyveromyces and can be selected for by resistance to G418 on regeneration plates containing glucose, sorbitol, and 0.2 µg/ml G418, while avoiding elevated concentrations of KC1, which interferes with the sensitivity of Kluyveromyces to G418. Preferred plasmids include the TRP1 gene, particulary from S. cerevisiae, the LAC4 gene, particularly from K. lactis the Kan^{R} gene providing for resistance against antibiotic G418 from Tn5, or the like.

The subject vectors and constructs are introduced into an appropriate cloning and expression of the desired structural genes. After transformation colonies will normally appear on regenertion medium within about 5 to 6 days. Where an antiobiotic is employed for selection, the colonies should be screend to ensure the absence of spontaneous mutation to a resistant strain. Employing the plasmids and the methods of the subject invention about 5% of resistant colonies were found to contain the plasmic construct providing for at least about 4 transformants per µg of plasmic DNA. Where selection was based on the presence of the LAC4 gene, using plates containing lactose as the sole carbon source and 0.6M KCl as an osmotic stabilizer, all of the surviving colonies were found to be transformants and not spontaneous revertants. About 20 transformants were obtained after about 4 to 5 days of incubation at moderate temperature, e.g. 30°C.

As a host organism, Kluyveromyces is especially suitable for the production of heterologous proteins, for example for the production and extration of the enzyme chymosin and its precursors preprochymosin, pseudochymosin and prochymosin, for human serum albumin (HSA), tissue plasminogen activator (t-PA) and thaumatin and its precursor forms. Although other organisms such as Saccharomyces produce prochymosin in reasonable amounts, the produced prochymosin cannot be extracted in an active or activatable form. We have surprisingly found that more than 90% of the total amount of the prochymosin produced by Kluyveromyces can be extracted in an active form with very simple standard techniques.

Any of the many Kluyveromyces species may be employed. Either laboratory or industrial, preferably industrial, strains may be used. By industrial species is intended, Kluyveromyces strains from organisms which may be isolated from natural sources or may be available from depositories or other sources or obtained by modification, e.g. mutation of such strains. The industrial strains are characterized by being resistant to genetic exchange, being prototrophic or made protorophic by a single gene being introduced into the host strain, and are usually selected for improved production of peptides. Among the Kluyveromyces species which may find use are K. lactis, K. fragilis, K. bulgaricus, K. thermotolerans, K. marxianus, etc. It should be further noted that the Kluyveromyces organisms are on the GRAS (Generally Recognized as Safe) list. Their use for production of products to be used in vivo or to be ingested normally will not require special governmental review and approval.

Both wild type and mutant Kluyveromyces particularly Kluyveromyces lactis or Kluyveromyces fragilis may be employed as hosts. Hosts of particular interest include K. lactis SD11 lac4 trp1 and K. lactis SD69 lac4, and the wild-type strain CBS 2360 (see EP-A-0096430).

For maintaining selective pressure on the transformants for maintenance of the plasmids, selective media may be used, such as a yeast nitrogen-based medium, 2% lactose instead of glucose for K. lactis SD69 lac4 (PTY75-LAC4) and for K. lactis SD69 lac4 (pL4) and a yeast nitrogen-based medium (Difco) plus 2% glucose for K. lactis SD11 lac4 trp1 (pKARS12). See for the transformants mentioned, EP-A-0096430. Similiarly, strains containing plasmics conferring antibiotic resistance for example against G418, may be cultivated in a medium containing said antibiotic.

Where the hybrid plasmids are employed for large scale production of the desired protein, it would generally be useful to remove at least substantially all of the bacterial DNA sequences from the hybrid plasmids.

For the secretion of the product both constitutive and non-constitutive transcriptional initiation regions may be employed depending on the fermentation process used for the production of the protein or polypeptide of interest. The expression product may be secreted into the culture medium, and produced on a continuous basis, where the medium is partially withdrawn, the desired product extracted e.g. by affinity chromatography, or the like, and the spent medium discarded or recirculated by restoring essential components.

All publications and patent applications mentioned in this specification are indicative of the level of skill of those skilled in the art to which this invention pertains. All publications and patent applications are herein incorporated by reference to the same extent as if each individual or patent application was specifically and individually indicated to be incorporated by reference.

The following examples are offered by way of illustration and not by limitation.

### EXPERIMENTAL

### Example 1

### Construction of chymosin expression plasmids containing a long lactase promoter sequence

### A. Construction of pUCla56

Chromosomal DNA was isolated from Kluyveromyces lactis strain CBS 2360 (Das and Hollenberg, Current Genetics (1982) 5:123-128), cleaved with Xhol, and separated according to size on a sucrose gradient. Fractions containing the lactase gene were detected with a LAC4 probe from plasrnid pK16 after spotting the DNA on a nitrocellulose filter (see EP-A-0096430, Example 16.C2). DNA containing the LAC4 gene was cloned into the Sall site of plasmid pPA153-215 (Andreoli, Mol. Gen. Genet. (1985) 199:372-380) giving rise to plasmid pPA31. An Xbal fragment of pPA31 containing the lactase gene was subcloned in the Xbal site of pUC19 (Yanisch-Perron et al., Gene (1985) 33:103-119) which yields plasmid pUCla56.

### B. Introduction of the G418 resistance gene in the terminator of the lactase gene

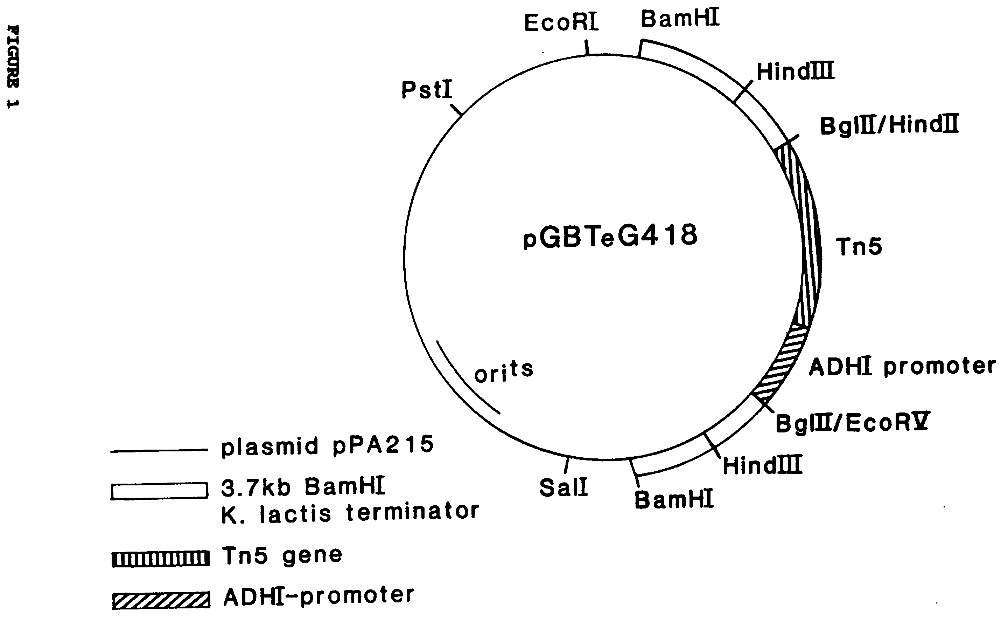

The terminator fragment containing the G418 resistance marker was obtained from plasmid pGBTeG418. E. coli containing pGBTeG418 was deposited with Centraal Bureau voor Schimmelcultures under number CBS 184.87 on February 26, 1987. Plasmid pGBTeG418 (see Figure 1) consists of the plasmid pPA153-215, as described above, and a 5.6 kb fragment consisting of the 3.7 kb BamHI K. lactis lactase terminator fragment (Breuning et al., Nucl. Acid Res. (1984) 12:2327-2341) and the Tn5 gene (Reiss et al., EMBO J. (1984) 3:3317) conferring resistance to G418 under the direction of the promoter alcohol dehydrogenase I (ADH) from yeast, similar to that described by Bannetzen and Hall, J. Biol. Chem. (1982) 257:3018-3025.

### C. Construction of plasmid pGB900 containing the G418 resistance gene and prochymosin encoding DNA

The 3.6 kb Hindlll-Xbal fragment from plasmid pGBTeG418 containing the G418 resistance gene (see Example 1B) and the Sall-Hindlll fragment containing the prochymosin gene from pGB123 (see EP-A-0096430) were ligated pUC19 cleaved with Sall and Xbal. This yielded plasmid pGB900.

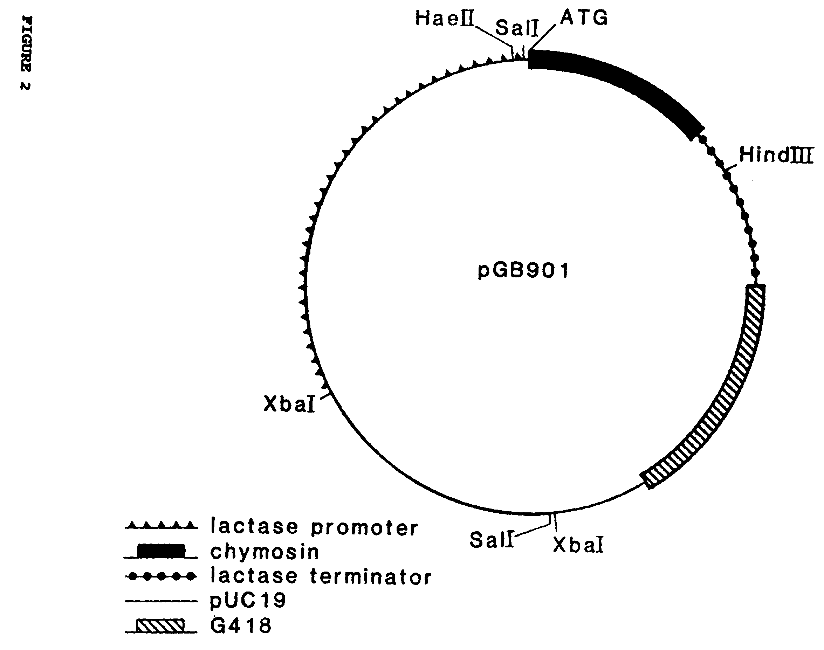

### D. Construction of plasmid pGB901 (see Figure 2)

Plasmid pGB901 was constructed by ligating the following four fragments:
(1) a 3.6 kb Xbal-Haell fragment containing the lactase promoter to about position -90 from the lactase ATG start codon isolated from puCla56.
(2) a Haell-Sall fragment extending from the above Haell site to a Sall site, which was ligated to position -26 in a similar Bal31 experiment as described in Example 16.C2 of EP-A-0096430. However, in this experiment only a Sall linker was used. This fragment has the following sequence. 
5' TTAAC ACTTGAAATT TAGGAAAGAG CAGAATTTGG CAAAAAAAAT AAAAAAAAAA TAAACACG 3'
3' CGCCGAATTG TGAACTTTAA ATCCTTTCTC GTCTTAAACC GTTTTTTTTA TTTTTTTTTT ATTTGTGCAG CT 5'
(3) the 5.1 kb Sall-Xbal fragment containing prochymosin and G418 from pGB900 (see Example 1C),
(4) pUC19 cleaved with Xbal.

During the construction of the plasmid the CG sequence from the Haell site was inadvertently removed, thereby creating a Hindlll site at this position.

Prochymosin-encoding DNA is present in plasmid pGB901. This may readily be converted to plasmids with proprochymosin. pseudochymosin or chymosin DNA by using the Sall-Bglll fragments from pGB 131. 122 or 124, respectively (see Ep-A-0096430).

### Example 2

### Secretion of prochymosin from Kluyveromyces lactis transformants

To direct the synthesis of prochymosin in Kluyveromyces, plasmid pGB901 was used to transform K. lactis strains SD11 and CBS 2360 with similar results. The transformation was carried out essentially as described in Examples 4 and 14 of EP-A-0096430, by using intact plasmid DNA or plasmid DNA cut with restriction endonucleases. In the latter case restriction endonucleases were used which cut in the promoter region, e.g., Sacll, Ndel, SnaBl or Spel, or in the terminator region, e.g., EcoRV, or both the promoter and terminator regions.

K. lactis strain CBS 2360 was grown in 100 ml of YEPD-medium (1% yeast extract, 2% peptone, 2% glucose) containing 2.5 ml of a 6.7% yeast nitrogen base (Difco) solution to an OD₆₁₀ of about 7. The cells were collected by centrifugation from 10 ml of the culture, washed with TE-buffer (10 mM Tris-HCl pH 7.5, 0.1 mM EDTA) and resuspended in 1 ml TE-buffer. An equal volume of 0.2 M lithium acetate was added and the mixture was incubated for 1 hr at 30°C in a shaking waterbath. Plasmid pGB901 (15 µg) was cut at the unique Sacll site in the lactase promoter. ethanol precipitated and resuspended in 15 µl TE-buffer. This DNA preparation was added to 100 µl of the pre-incubated cells and the incubation was prolonged for 30 minutes. Then an equal volume of 70% PEG 4000 was added and the mixture was incubated for 1 hr at the same temperature, followed by a heatshock of 5 minutes at 42°C. Then 1 ml of YEPD-medium was added and the cells were incubated for 1.5 hrs in a shaking waterbath of 30°C. Finally the cells were collected by centrifugation, resuspended in 300 µl YEPD and spread on agar plates containing 15 ml of YEPD agar with 300 µg/ml of G418, over-layered (1 hr before use) with 15 ml YEPD-agar without G418. Colonies were grown for 3 days at 30°C. K. lactis strain SD11 was transformed in a similar way, only the initial G418 concentration in the selection plates was lowered to 150 µg/ml. In one of the experiments transformants of CBS 2360 were grown at 30°C in YEP-medium containing 2% galactose. After 60 hours, cells and medium were separated by centrifugation. Cells were disrupted by treatment with glass beads. Culture medium and cell extract were treated at pH 2 before assaying for chymosin activity (see Foltman. Methods in Enzymology (1970) 19:421-426).

Cells were removed from cultures by centrifugation and the resulting supernatants were acidified to pH 2 by the addition of 1 M H₂SO₄ and incubated for 2 hours at room temperature. The solutions were then neutralized to pH 6 by the addition of 2 M Tris base. A 50 µl volume of an appropriate dilution was added to a suspension of 12% non-fat dry milk in 10 mM CaCl₂ and incubated at 37°C until a clot formed. A unit of chymosin activity is defined as the amount of active chymosin required to produce a clot in 10 min, under these conditions. The supernatant contained milk-clotting activity due to the production and secretion of prochymosin by K. lactis transformants although no signal sequence for protein secretion was added to prochymosin. About 30-60% of the total prochymosin produced was found in the medium as determined by the above-described milk-clotting assay. Similar results were obtained when K. lactis strain SD11 was used.

### Example 3

### Lactase-chymosin fusion proteins giving enhanced chymosin production

By taking various SnaBl-Sall fragments (from a Bal31 experiment similar to the one described in Example 16.C2 of EP-A-0096430 but using a single Sall linker only) variants of pGB901 containing a fusion between the lactase and chymosin proteins were obtained (Table 1). The extra amino acids provided by lactase DNA and linker sequences con be removed, along with the pro sequence of prochymosin, by treatment with acid. It was observed that a fusion containing 4 amino acids from the lactase coding sequence (pGB902) resulted in enhanced chymosin production.

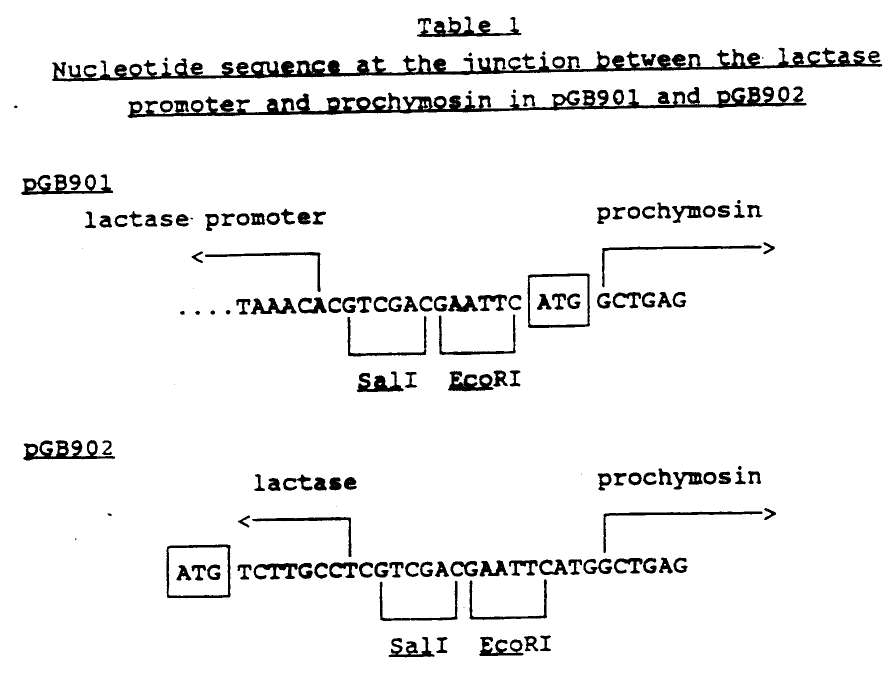

Protein synthesis starts at the boxed ATG codon.

### Example 4

### Expression of preprochymosin by Kluyveromyces transformants

The Sall site from the polylinker of pGB902 (see Example 3) was removed for convenience. pGB902 was partially digested with Sall, followed by a short incubation with Bal31 (Boehringer). Linear fragments were isolated from an agarose gel, ligated and transformed into E. coli. A correct plasmid. pGB903, was obtained. Restriction analysis showed that this plasmid also has the Xbal and Hindlll sites removed from the polylinker.

To construct a plasmid containing and expressing preprochymosin, plasmid pGB903 was digested with the restriction endonucleases Sall and Bglll. The 11 kb DNA fragment was isolated from an agarose gel by electroelution. Similarly, plasmid pGB124 containing the preprochymosin gene (see EP-A-0096430. Example 16) was digested and the 0.3 kb SallBglll fragment containing the N-terminal part of the preprochymosin gone was isolated.

The 11 kb and the 0.3 kb DNA fragments were mixed, ligated with DNA ligase and transformed into E. coli. Plasmid pGB904 was isolated which contained the preprochymosin gene fused to a small part of the lactase gene (Table 2).

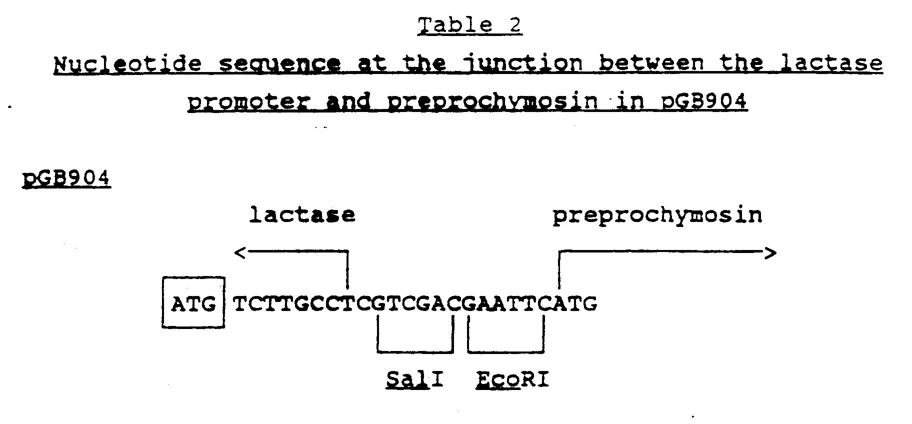

Protein synthesis starts at the boxed ATG codon.

K. lactis CBS 2360 cells were transformed with pGB904. which had been linearized with Sacll. Transformants were selected, grown and assayed for chymosin activity as described in Example 2. In the following Table 3 a comparison is made between the secretion of prochymosin from K. lactis CBS 2360 cells transformed with pGB902 (see Example 3) and with pGB904. (Pro)chymosin production is expressed in arbitrary units per ml of cells at OD₆₁₀ of 200.

**Table 3**

| Secretion of prochymosin by K. lactis cells transformed with pGB902 and pGB904 | | | | |
|---|---|---|---|---|
| Transformant | pGB902 | | pGB904 | |
| | Supernatant | Pellet | Supernatant | Pellet |
| 1 | 3.2 | <0.4 | 22.4 | 1.7 |
| 2 | 1.3 | <0.4 | 33.3 | 3.0 |
| 3 | 7.1 | 1.4 | 28.0 | 2.3 |
| 4 | 4.4 | 0.66 | 53.8 | 5.8 |

### Example 5

### Secretion of prochymosin by Kluyveromyces using heterologous leader sequences

### A. Chemical synthesis of an amyloglucosidase leader sequence and construction of a plasmid containing said leader sequence

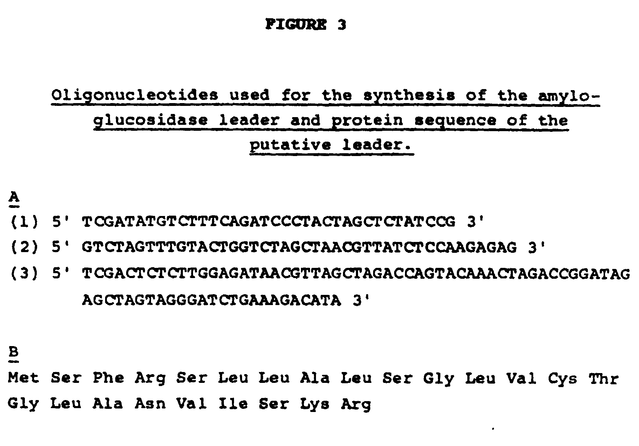

The leader sequence of amyloglucosidase (AG) from Aspergillus awamori was published by Innis et al., Science (1985) 228:21-26. Based on the protein sequence, oligonucleotides were derived to permit insertion of the leader sequence in front of the prochymosin gene (see Figure 3).

The oligonucleotides were synthesized with an Applied Biosystems DNA synthesizer. The oligonucleotides were purified by electrophoresis on a denaturing polyacrylamide gel, then electroeluted from the gel.

Plasmid pGB903 (see Example 4) was cut at the unique Sall site. The oligonucleotides were hybridized at 65°C. 50°C and 37°C for one hour each in 2xSSC. The oligonucleotides had no phosphate at the 5' end to prevent formation of multimers. The DNA was ligated into the Sall site using T4 polynucleotide ligase. The ligation mixture was transformed into E. coli HB101. Twenty-Tour of the colonies were cultured and plasmid DNA isolated. One of the plasmids pGB905 was shown to have the correct orientation of the oligonucleotides by restriction enzyme analysis. Plasmid pGB905 was transformed to K, lactis CBS 2360. (Pro)chymosin production was analyzed according to the procedure described in Example 2. The results are of the (pro)chymosin production, in arbitrary units/ml of cells a: OD₆₁₀ of 200, is shown in the following Table 4.

**Table 4**

| Secretion of prochymosin by K. lactis cells transformed with pGB902 and pGB905 | | | | |
|---|---|---|---|---|
| Transformant | pGB902 | | pGB905 | |
| | Supernatant | Pellet | Supernatant | Pellet |
| 1. | 3.2 | <0.4 | 60.6 | <0.4 |
| 2. | 1.3 | <0.4 | 56.4 | <0.4 |
| 3. | 7.1 | 1.4 | 56.7 | <0.4 |
| 4. | 4.4 | 0.66 | 57.6 | <0.4 |

### B. Chemical synthesis of a novel synthetic leader sequence into construction of a plasmid containing the novel synthetic leader sequence.

A synthetic leader sequence was prepared which has a sequence different from any known leader sequence. Using this leader sequence, all prochymosin synthesized was secreted by Kluyveromyces as shown below.

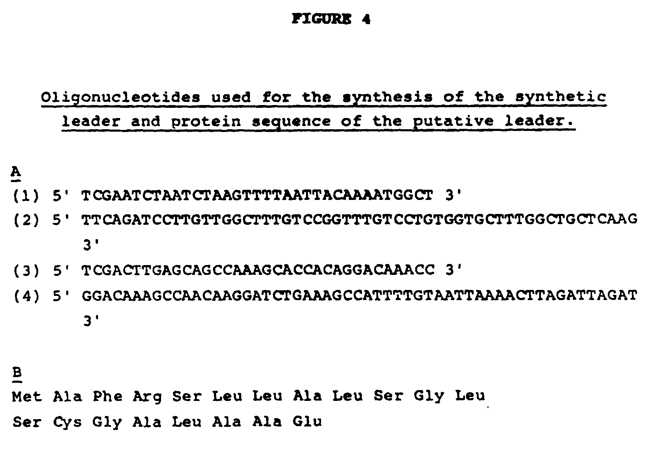

This synthetic leader sequence was devised using frequently occurring amino acids from position -6 to +2 of the signal sequence cleavage site (Von Heyne, Eur. J. Biochem. (1983) 133:17-21). Frequently occurring yeast codons were also employed and extra nucleotides were incorporated in front of the ATG sequence to make up for the deletion of 26 nucleotides in pGB902. The oligonucleotides used and the resulting leader sequence are shown in Figure 4.

The synthetic leader sequence DNA was synthesized using an Applied Biosystems DNA synthesizer. The resulting oligonucleotides were run on a 40 cm long, 1 mm thick polyacrylamide gel. containing TBE buffer (50 mM Tris, 50 mM borate, 1mM EDTA, pH 8.3) and 7 M urea until the Bromophenol Blue marker had travelled 2/3 of the gel length. The DNA was visualized, eluted from the gel and precipitated with ethanol.

Also from pGB901 a derivative was made with a deletion around the Sall site resulting from the polylinker of pUC19. This was done by replacing the 0.5 kb SnaBl-Bglll fragment from pGB903 by the corresponding fragment from pGB901. The resulting plasmid was cut at the unique Sall site. The oligonucleotides were hybridized at 65°C, 50°C and 37°C for one hour each in 2xSSC. The DNA was ligated into the Sall site using T4 polynucleotide ligase. The plasmid was then transformed into E. coli HB101. Of the colonies obtained, 24 were cultured and plasmid DNA isolated. One of the plasmids, pGB906, was shown to have the oligonucleotides in the correct orientation by restriction enzyme digestion. It was found that K. lactis CBS 2360 transformed with pGB906 secreted more than 95% of the prochymosin produced.

### C. Analysis of chymosin protein produced by K. lactis transformed with pGB905

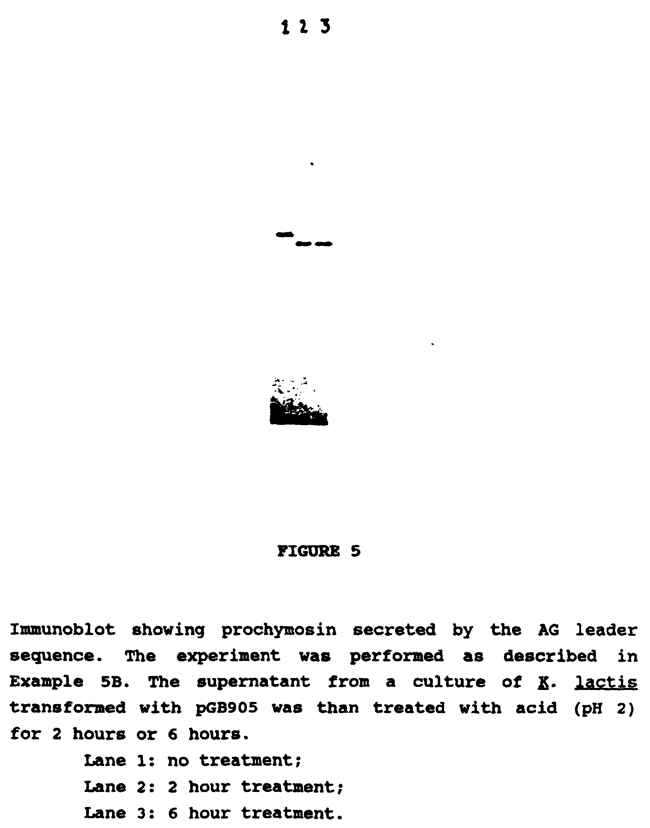

K. lactis CBS 2360 (pGB905) transformants were grown for 3 days at 30°C and samples were collected from the supernatant of the cultures. Protein samples were electrophoresed on a poIyacrylamide gel according to Laemmli (Nature (1970) 227:680-685). Proteins were blotted onto a nitrocellulose filter according to the method of Towbin et al. (Proc. Natl. Acad. Sci. USA (1979) 76:4350-4354). Chymosin protein was detected by incubating the filter with a polyclonal antiserum against chymosin (Chr. Hansen), followed by donkey anti-rabbit antibodies coupled to a peroxidase (Amersham) and finally with 0.6 mg/ml 4-chloronaphthol and 0.015% hydrogen peroxide in a buffer solution (50 mM Tris-HCl pH 7.5. 0.9% NaCl) containing 40% methanol. Prochymosin excreted by the AG signal sequence is correctly cleaved after pH 2 treatment as demonstrated by this assay (Figure 5). Similar results were obtained with K. lactis CBS 2360 (pGB906) transformants.

### Example 6

### Construction of plasmids containing the Saccharomyces cerevisiae α-factor sequence or efficient secretion

### A. Saccharomyces α-factor expression plasmids

### 1. Construction of Plasmids

pDM100-PC: The starting material was plasmid pGB163 (see EP-A-0096430, Example 16.C1). Plasmid pGB163 was digested with BamHl and ligated to an Xbal-BamHl, α-factor leader-prochymosin adaptor. The resulting mixture was then treated with Pstl and a 96 bp fragment encoding the pro-α-factor processing site and the N-terminal region of prochymosin was isolated. A 1900 bp fragment encoding bovine prochymosin was isolated from plasmid pJS111 following digestion with Pstl and Sall. Plasmid pJS111 is a pBR322 derivative containing the prochymosin gene from pGB163 under the regulatory control of the ADH-2 promoter and the glyceraldehyde 3-phosphate (GAPDH) terminator. The 1900 bp Pstl to Sall fragment that was removed contains the prochymosin gene and the GAPDH terminator. The yeast GAPDH 49 gene promoter and transcription terminator are essentially as described by Travis, J. Biol. Chem. (1985) 260:4384-4389.

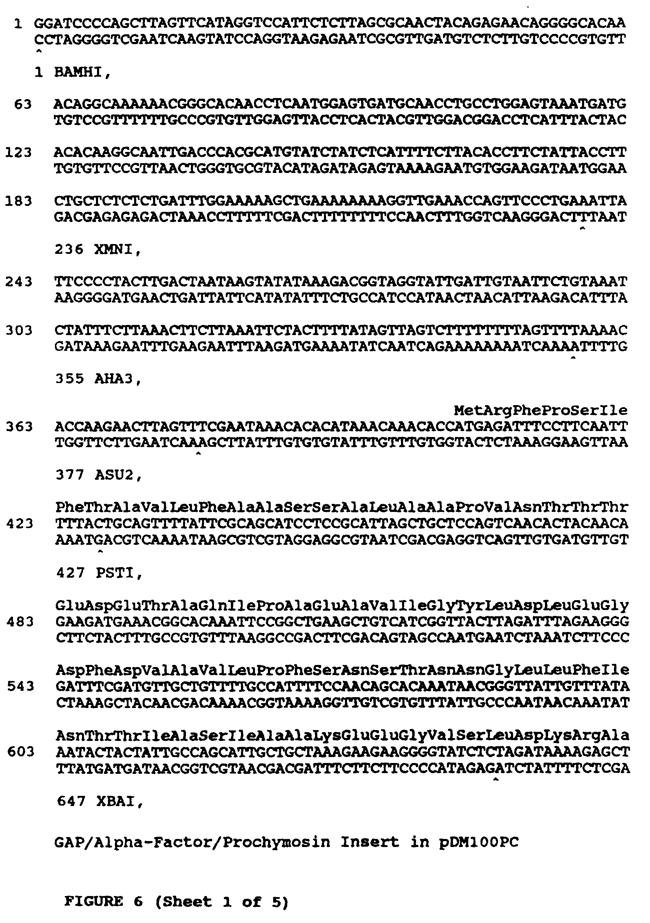

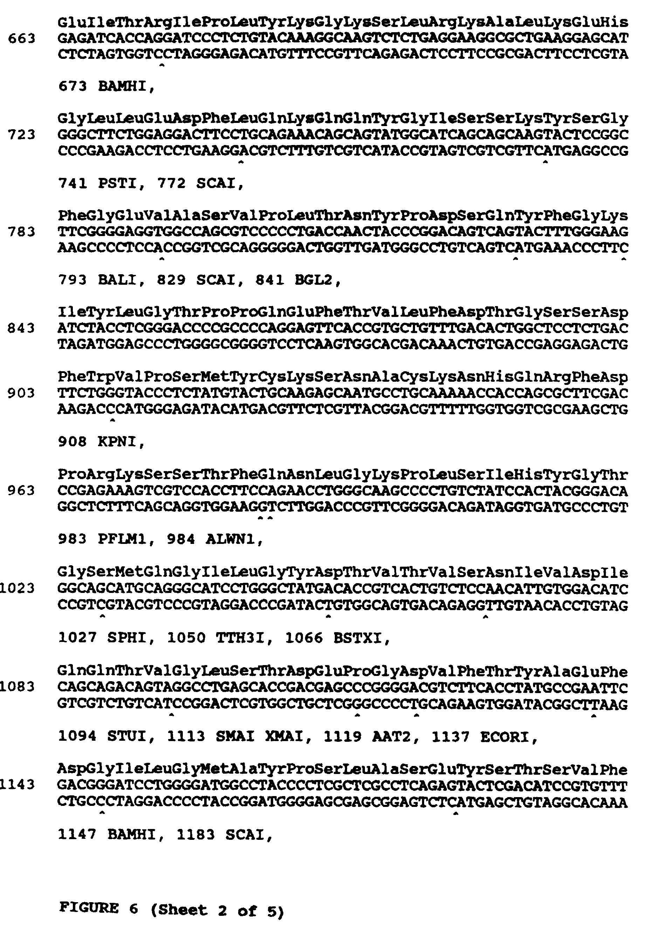

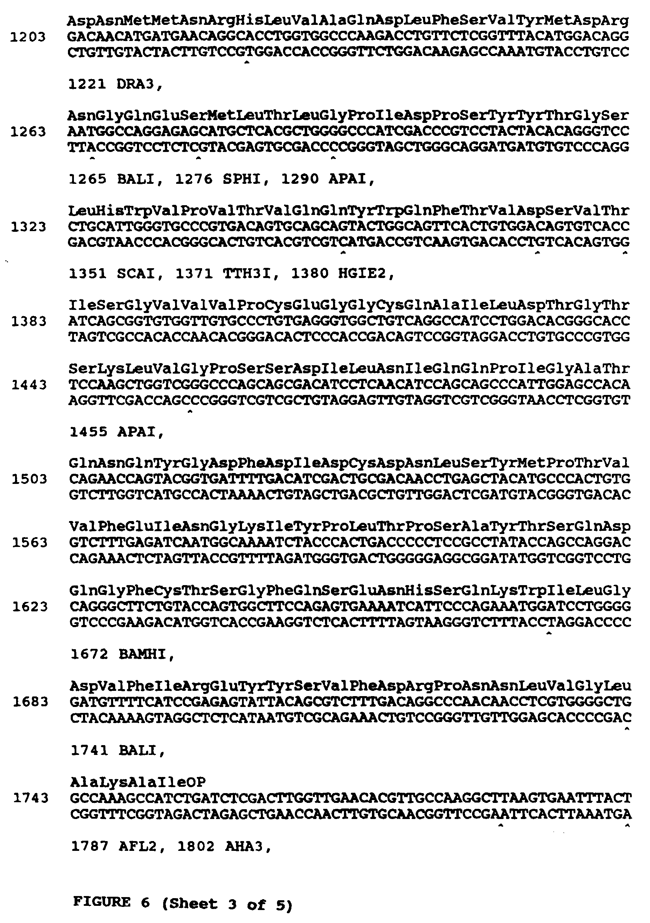

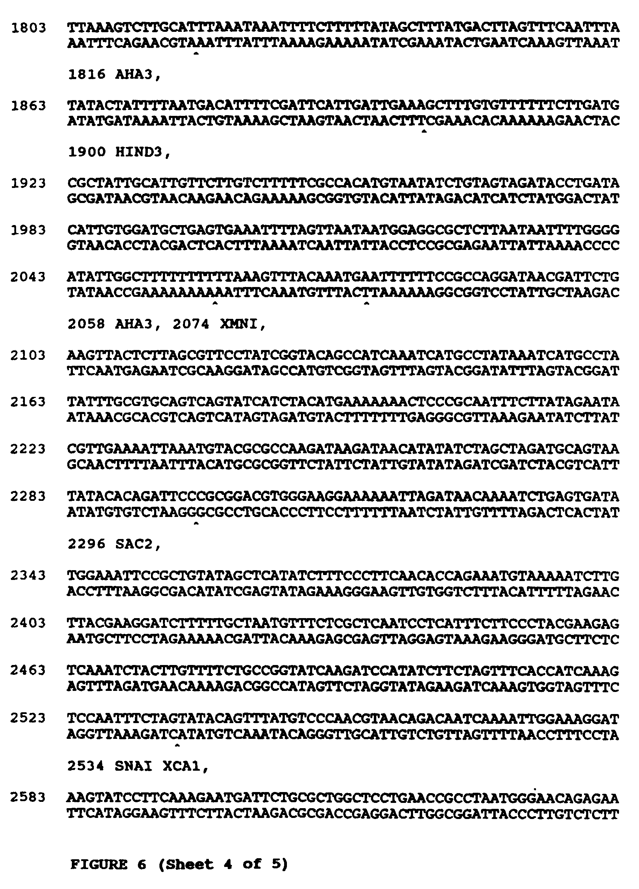

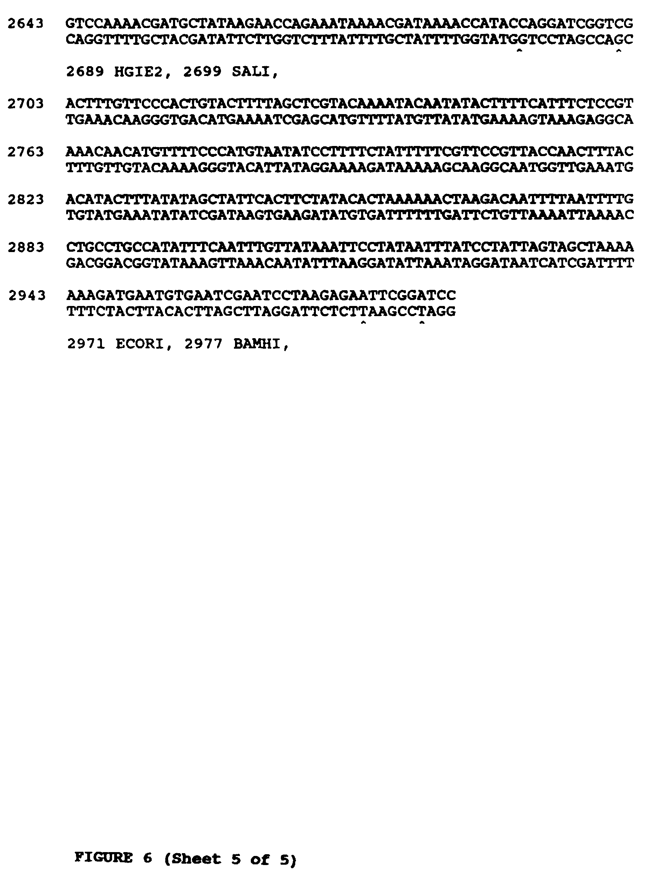

Plasmid pDM100, containing a fusion of the GAPDH promoter, the S. cerevisiae α-factor leader, and a synthetic gene for human γ-interferon flanked by Xbal and Sall sites and the α-factor terminator, was digested with Xbal and Sall, treated with alkaline phosphatase, then ligated to the 96 bp and 1900 bp fragments described above. The α-factor leader and terminator are essentially as described by Brake, Proc. Nat. Acad. Sci. USA (1984) 81:4642-4646. The resulting plasmid pDM100-PC was isolated and contained a fusion of the GAPDH promoter, the α-factor leader and prochymosin gene. The complete sequence of the BamHl insert is shown in Figure 6.

To allow selection of yeast transformants, two plasmids, pKS100 and pAB300, were constructed.

pKS100: Plasmid pKS100 was constructed by insertion into pDM100-PC of an 1170 bp Hindlll fragment from YEp24 containing the S. cerevisiae URA3 gene.

pAB300: Plasmid pAB300 was constructed by insertion into pDM100-PC of a 3500 bp Hindlll-Sall fragment from pGB901 containing the 3' region of the K. lactis LAC4 gene and the G418 resistance marker. The GAPDH/α-factor/prochymosin Baml insert in pDM100-PC is illustrated in Figure 6.

### 2. Transformation of K. lactis and S. cerevisiae

Plasmid pKS100 was digested at the Bglll site in the prochymosin coding region and used to transform K. lactis strain KRN201-6. This strain is a derivative of strain 2UV21 (a lac4 trp1 ura3 [kil°]) in which the lac4 gene has bean replaced by the LAC4 promoter-prochymosin gene fusion from pGB901. Integration of pKS100 is thus targeted to the integrated prochymosin coding region. Plasmid pKS100 was also used to transform S. cerevisiae strain AB110 (α ura3 leu2 his4 pep4-3 [cir°]), in this case targeting to the Sacll site in the 3' region of the GAPDH gene.

The resulting transformants were grown to saturation in liquid YEPD medium, and the culture supernatants and cell lysates assayed for chymosin activity after activation at pH 2. As shown by the results summarized in Table 5 below, the K. lactis transformants efficiently secreted prochymosin into the medium, whereas the S. cerevisiae transformants secreted only a small fraction of the prochymosin produced.

**Table 5**

| Prochymosin production in K. lactis and S. cerevisiae transformants | | |
|---|---|---|
| Strain | Chymosin Activity (relative units/ml culture) | |
| | Cell Extract | Culture Supernatant |
| AB110 | <0.25 | <1.0 |
| AB110::pKS100 | 15.5 | 2.3 |
| KRN201-6 | <0.25 | <1.0 |
| KRN201-6::pKS100 | 12.0 | 333.0 |

Plasmid pAB300 was used to transform K. lactis strain 2UV21 to G418 resistance, targeting integration to the EcoRV site in the 3' region of the LAC4 gene. These transformants were also found to efficiently secrete prochymosin into the culture medium as shown in Table 6 below.

**Table 6**

| Prochymosin secretion from α-factor/prochymosin fusions | | |
|---|---|---|
| Host Strain | Transforming Plasmid | Secreted Chymosin Activity (relative units/ml culture) |
| 2UV21 | - | <2 |
| KRN201-6 | - | <2 |
| KRN201-6 | pKS100 | 385 |
| 2UV21 | pAB300 | 294 |

### B. Construction of LAC4 promoter/α-factor leader/prochymosin fusions

In order to produce this fusion, two intermediate plasmids were constructed. Plasmid pDM100-PC was partially digested with Pstl, ligated to a Sall-Pstl adaptor encoding a portion of the α-factor leader and 26 bp of the region 5' to the LAC4 gene, and then digested with Hindlll. A 1500 bp fragment was isolated from this mixture and then cloned into pUC18 digested with Hindlll and Sall to produce pKS102.

A synthetic E. coli lac operator was ligated into the Sall site just 5' to the α-factor leader coding sequence in pKS102 to produce the plasmid pKS103. This was done because the LAC4 promoter/α-factor leader/prochymosin fusion may be toxic to E. coli.

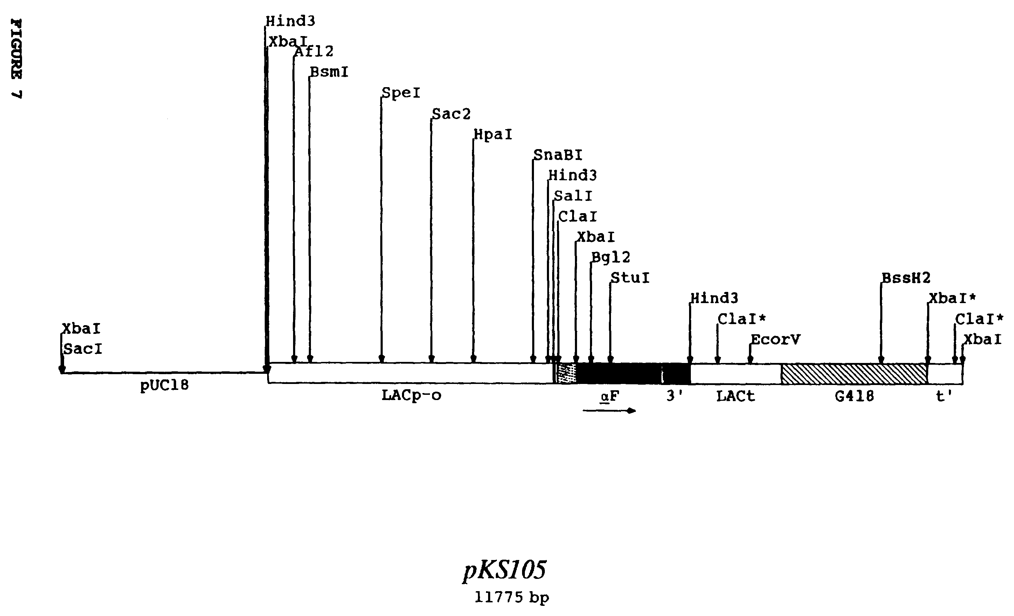

A 490 bp Sall-Bglll fragment from pKS103 was isolated and ligated to Sall-Bglll-digested pJD15R. Plasmid pJD15R is derived from pGB901 by deletion of the Sall site in the pUC19 poylinker by filling-in to produce pJD15, and then recloning the 8800 bp Xbal fragment in the opposite orientation. From this reaction the plasmid pKS105 was isolated. These plasmids are illustrated in Figure 7.

Plasmid pKS105 was then used to transform K. lactis strain CBS 2360 to G418 resistance, using the Sacll site in the LAC4 5' region as a targeting site for the integrative transformation. Chymosin production is expressed in units per ml of cells at OD₆₁₀ of 200, see Table 7 below.

**Table 7**

| Secretion of Prochymosin by K. Lactis Cells Transformed with pKS105* | | |
|---|---|---|
| Transformant | Supernatant | Pellet |
| 1 | 111 | 3.3 |
| 2 | 147 | 4.5 |
| 3 | 124 | 3.7 |
| 4 | 125 | 3.0 |

| | | |
|---|---|---|
| *Chymosin activity in relative units/ml culture. | | |

### Example 7

### Construction of plasmids containing the Kluyveromyces lactis α-factor sequence for efficient secretion

### A. Isolation and use of K. lactis α-factor signal sequence

Biological assays of culture supernatants were carried out as described (Julius. et al., Cell (1983) 32:839) using as a tester strain the S. cerevisiae Mat a sst2-3 strain RC687. K. lactis strain CBS 141 (α) was grown in a medium consisting of 0.5% glucose, 0.17% yeast nitrogen base without ammonium sulfate (Difco), and 0.002% ammonium sulfate. After removal of cells by centrifugation, acetic acid was added to the culture supernatant to a concentration 0.1 M, and supernatant was passed over a column of Bio Rex 70 (Biorad). The column was washed with 0.1 M acetic acid and then the α-factor was eluted with 80% ethanol/10 rnM HCl. The eluate was evaporated to dryness and then dissolved in 0.1% trifluoroacetic acid (TFA)/20% acetonitrile and applied to a reverse-phase HPLC guard column. The column was washed stepwise with solutions containing 0.1% TFA and 20%, 40%, 60%, and 80% acetonitrile. The 60% fraction, containing the α-factor activity, was then applied to an analytical C-18 HPLC column and eluted with a gradient of 20% to 80% acetonitrile in 0.1% TFA. Fractions were collected and assayed for α-factor activity. The fractions containing α-factor activity wee dried and subjected to amino acid sequence analysis.

### B. Hybridization screening of plasrnid libraries

Pools of oligonucleotides were labeled using γ- [³²P]-ATP and T4 polynucleotide kinase. These oligonucleotide probes were used to probe Southern blots or bacterial colonies at 42°C in the following hybridization solution: 4xSSC, 50 mM KH₂PO₄ pH 7, 1% sarkosyl, 10% dextran sulfate, 200 µg/ml sonicated, denatured salmon sperm DNA. Filters were washed in 2xSSC, 0.1% SDS at 42°C.

A plasmid library in the vector pJS109, containing inserts resulting from a limited Sau3Al digest of genomic DNA from K. lactis strain SD11 (a trp1 lac4), size-fractionated to purify fragments >5000 bp was screened with these-probes by plating transformants of E. coli strain HB101 at a density of 500-2000 colonies per 80 mm plate of L-agar containing 100 µg/ml ampicillin. DNA was transferred from the colonies to nitrocellulose filters and these filters hybridized as described above. Areas on the original plates corresponding to regions of hybridization signals on the filters were picked, then replated and retested by hybridization to isolate single colonies with plasmids containing hybridizing sequences. Positive colonies were further tested by Southern blot analysis of DNA purified from small cultures.

Plasmids purified from hybridization-positive colonies were digested with a variety of restriction enzymes and the resulting fragments analyzed by Southern blot analysis using the same hybridization probes in order to identify restriction fragments of size suitable for DNA sequence analysis. Fragments thus identified were purified by agarose gel electrophoresis and cloned into appropriate MP18 and MP19 vectors. DNA sequence analysis was then performed.

### C. Isolation of Kluyveromyces α-factor

The first 10 amino acids of the K. lactis α-factor showed a definite homology to that from S. cerevisiae, with 3 identical residues. This sequence is shown below:
Trp-Ser-Trp-lle-Thr-Leu-Arg-Pro-Gly-Gln

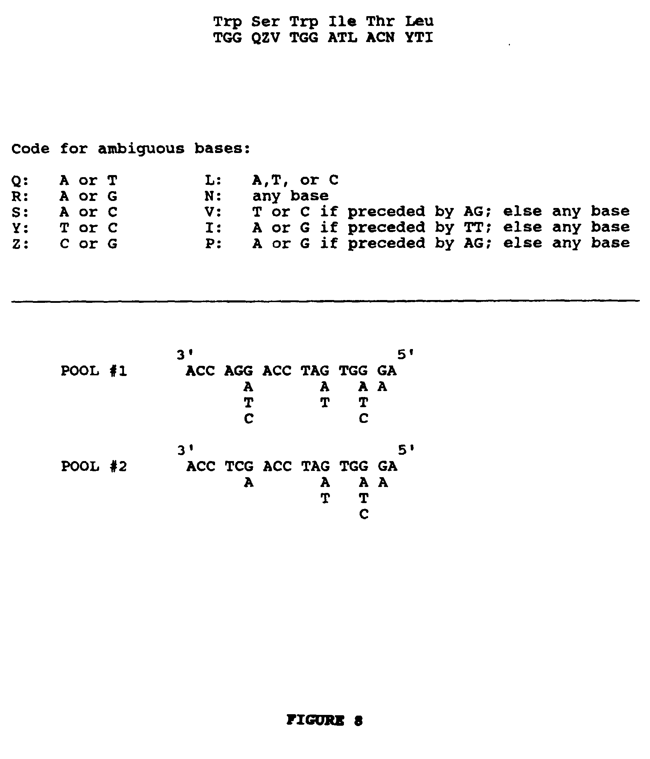

This protein sequence gas used to design a set of oligonucleotides deduced to be complementary to the structural gene for the corresponding structural gene as shown in Figure 8. Oligonucleotides including all of the possible codons for a segment of the α-factor peptide where synthesized as two pools of 96 and 48 different molecules.

These two pools here radioactively labeled using γ-[³²P]-ATP and T4 polynucleotide kinase, and were each used to probe a Southern blot of restriction digests of K. lactis DNA. Pool #2 gave strong hybridization to a single fragment and much weaker hybridization to a second fragment in several different digests. Thus, pool 2 was chosen to screen plasmid libraries of K. lactis genomic DNA.

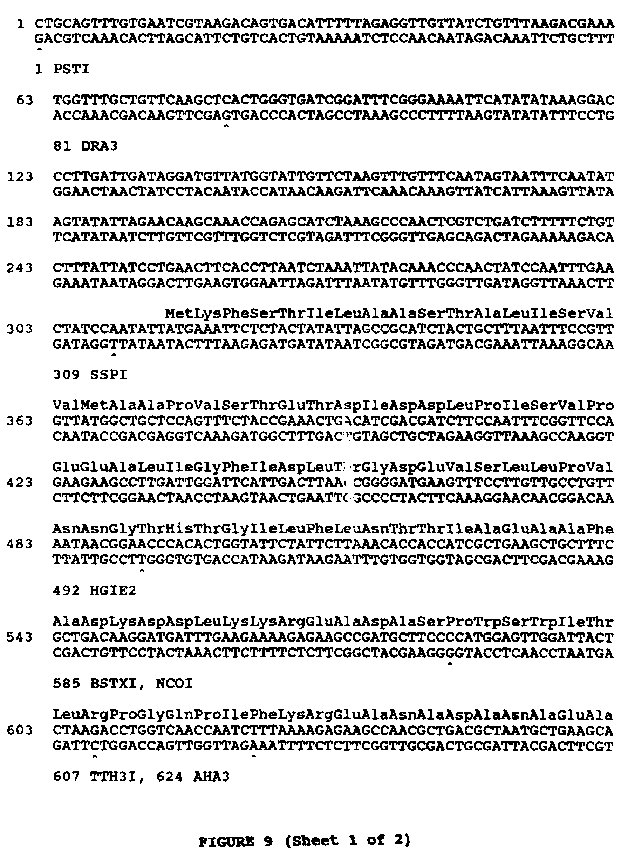

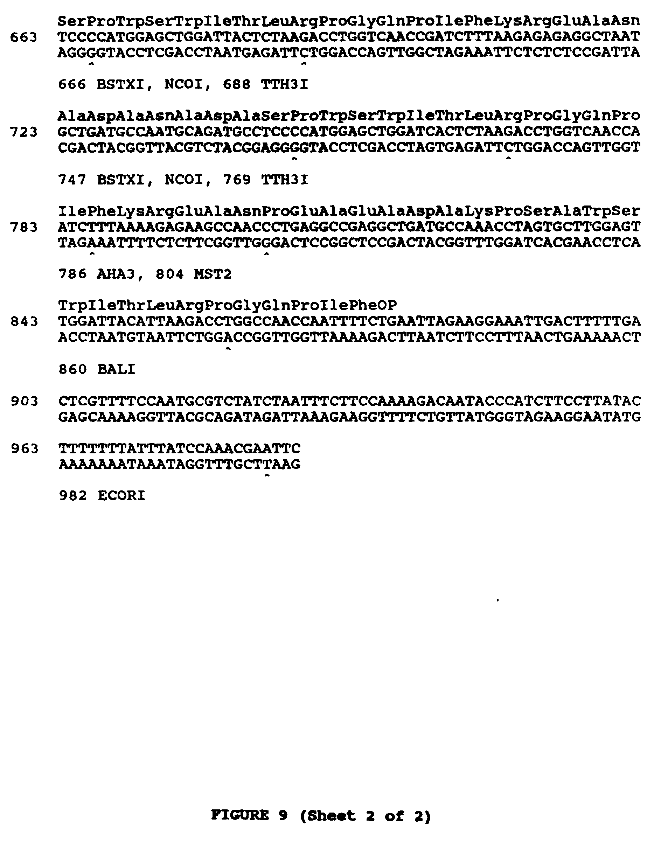

Use of these probes to screen plasmid libraries resulted in the isolation of a number of hybridizing clones. DNA sequence analysis of one of these clones, αfk18b, showed it encodes an α-factor related peptide which bears a strong similarity to the precursor of the S, cerevisiae α-factor peptide. The hybridizing segment was located on a Pstl-EcoRl fragment of about 1000 bp. The sequence of this fragment is shown in Figure 9. The K. lactis precursor contains only 2 sites for the addition of N-linked carbohydrate chains. In addition, the spacers of the K. lactis repeats are longer than those of the S. cerevisiae repeats and show a more diverse sequence with the pattern X-Ala/Pro rather than the Glu/Ala-Pro sequences found in S. cerevisiae. A comparison of the DNA sequences showed a strong degree of homology throughout the coding region.

### D. Construction of Plasmids

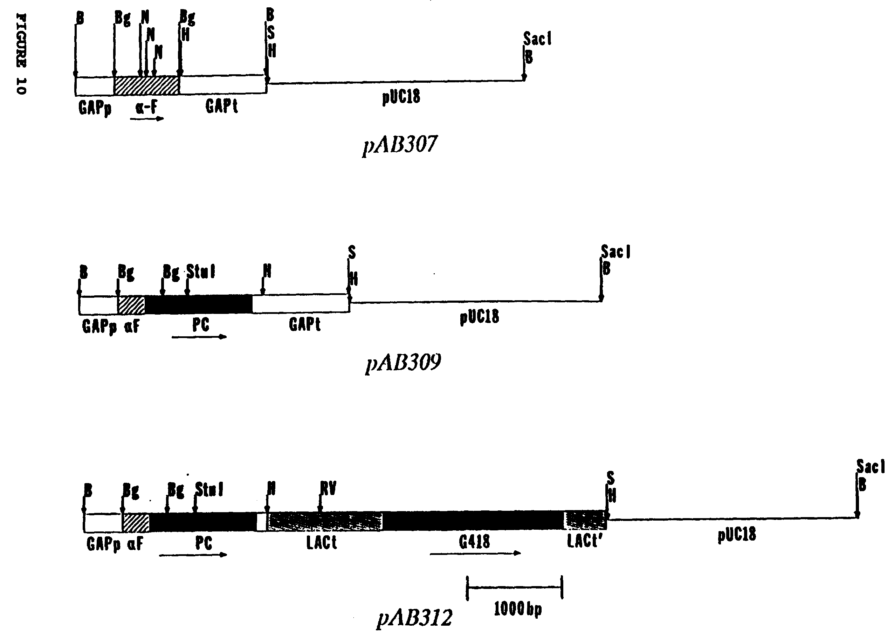

A series of plasmids (shown in Figure 10) where constructed in order to provide a fusion of the K. lactis α-factor leader to prochymosin expressed under the transcriptional control of a strong promoter.

pAB307: A 673 bp Ssol-EcoRl fragment from αfk18b (Figure 9) was modified by filling the EcoRl overhang by Klenow enzyme and addition of Bglll linkers to the blunt ends. This fragment was then inserted into a Bglll site joining the promoter and terminator regions of the S. cerevisiae glyceraldenyde-3-phosphate dehydrogenase gene (GAPDH). This cassette was cloned as a BamHl fragment in pUC18, resulting in pAB307.

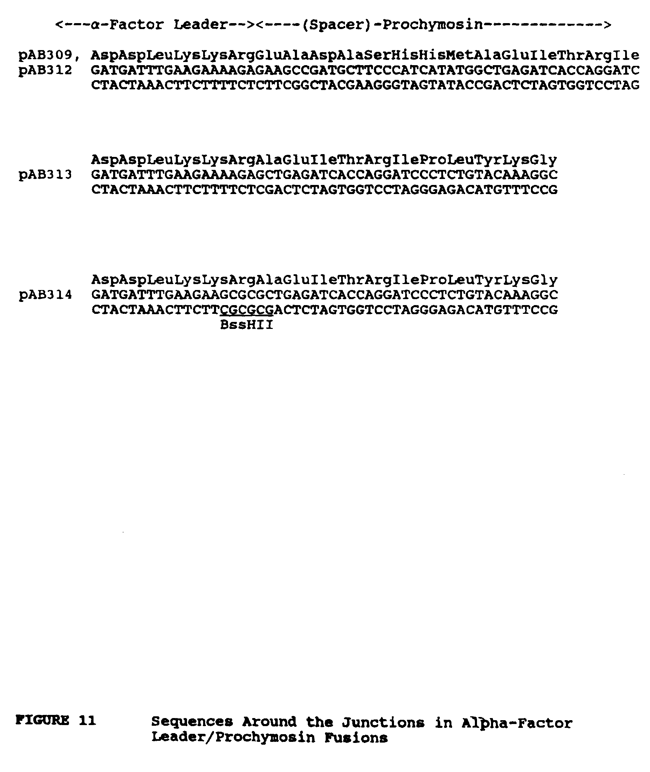

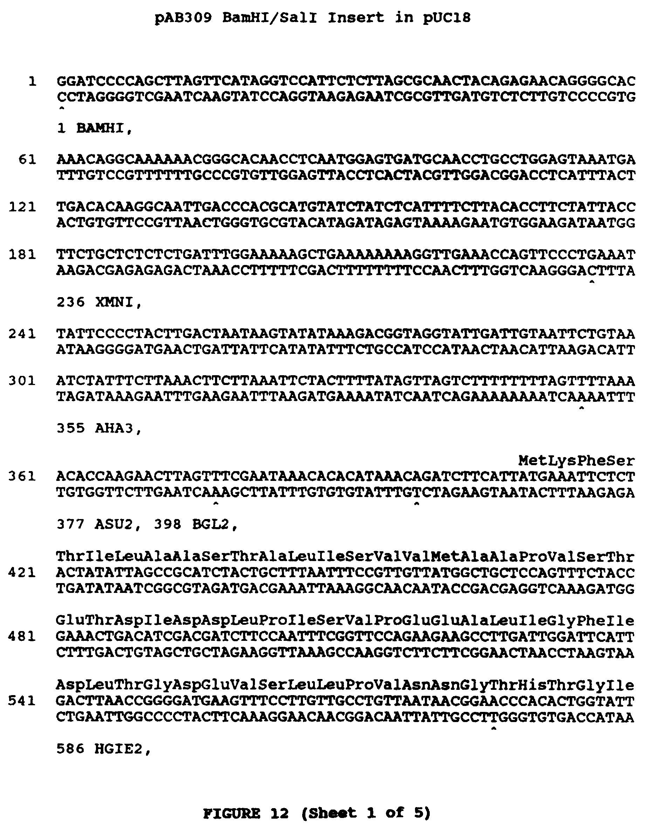

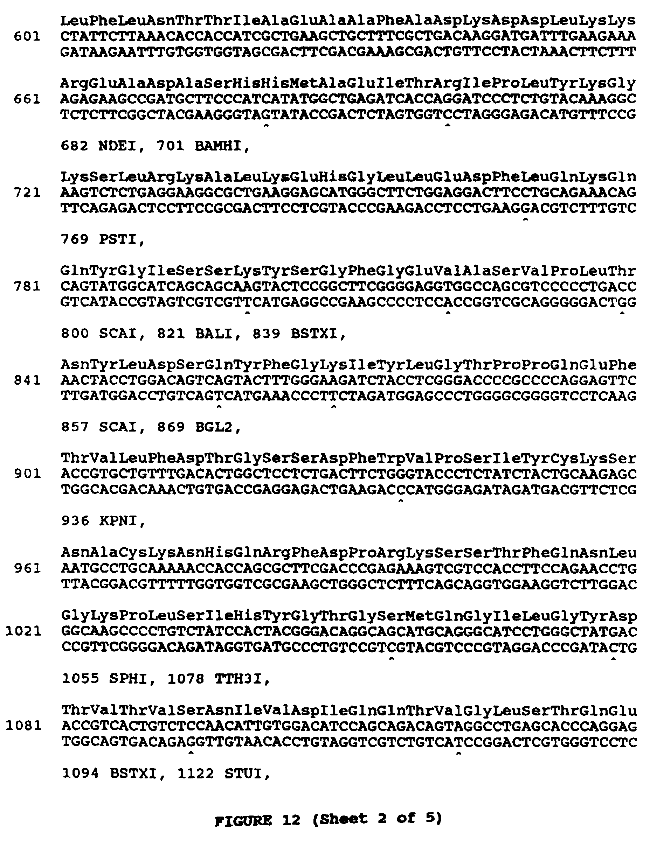

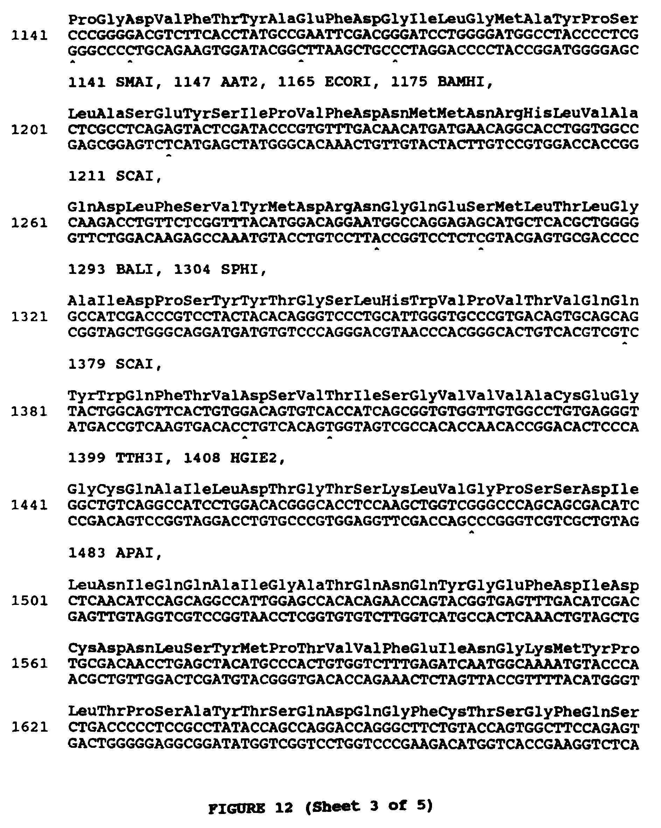

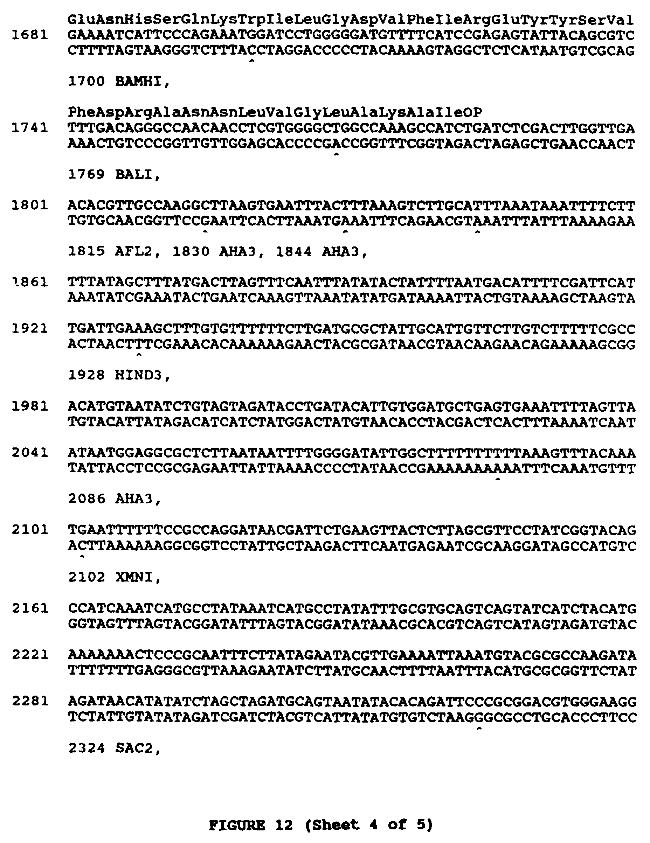

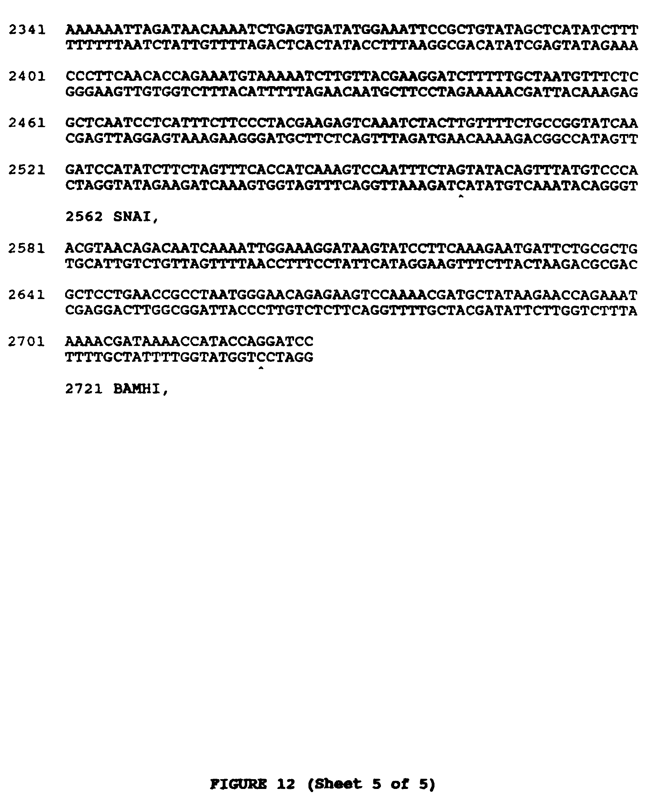

pAB309: Fusion of sequences encoding the α-leader and bovine prochymosin was then performed. First pAB307 was digested with Ncol and the cohesive ends made blunt by treatment with mung bean nuclease. The resulting product was then digested with Sall. To this was ligated a 2000 bp EcoRV-Sall fragment containing sequences encoding prochymosin and the S. cerevisiae transcriptional terminaion region. This fragment was derived from plasmid pJS111 in which Xbal-BamHl adaptor had been added to the 5' end of a fragment containing prochymosin cDNA fused to the S. cerevisiae GAPDH transcriptional termination region. This ligation mixture was used to transform E. coli strain HB101 and a transformant carrying the plasmid pAB309 was isolated. The sequences around the junction of this fusion are shown in Figure 11 and the sequence of the entire BamHl-Sall insert of pAB309 is shown in Figure 12.

pAB312: In order to obtain transformation of K. lactis strains. a 3560 bp Hindlll fragment derived from pGB901 was inserted into pAB309 producing plasmid pAB312. The Hindlll fragment contains the 3' region of the K. lactis LAC4 gene and a fusion of the S. cerevisiae ADH1 promoter to the bacterial G418-resistance structural gene.

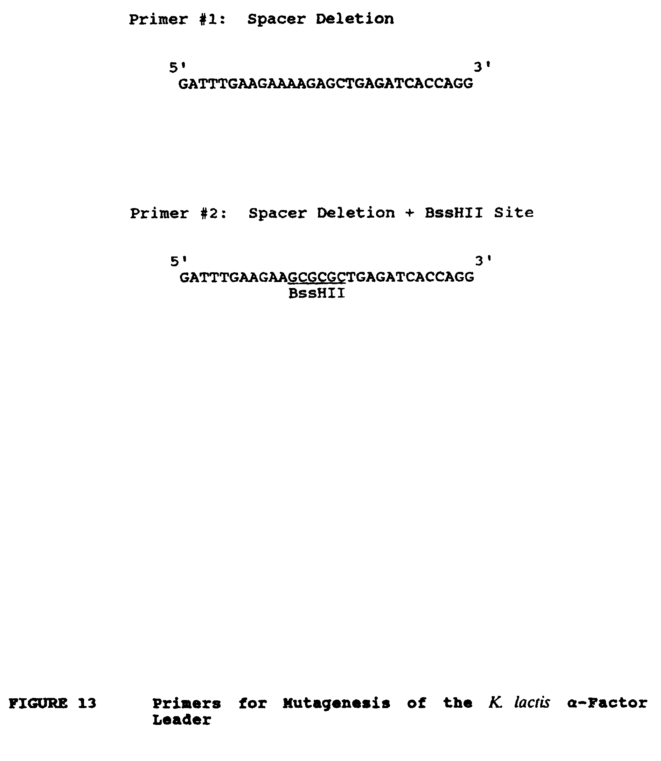

pAB313 and pAB314: A 1900 bp Sacl-Hindlll was isolated from pAB309 and cloned into MP19 (Yanisch-Perron et at.. Gene (1985) 33:103). Single-stranded phage DNA was prepared and used as a template for in vitro mutagenesis with one of the two oligonucleotide primers shown in Figure 13. The M13 phage MP19/αk11.5 and MP19/αk12.2 were prepared using Primer #1 and Primer #2, respectively.

Double-stranded RF DNA was prepared from these phages, and 1100 bp Sacl-Stul fragments isolated from each. These fragments were ligated to a 7100 bp Sacl-Stul fragment from pAB312. The resulting plasmids pAB313 and pAB314 were isolated with the sequence alterations illustrated in Figure 13.

### E. Transformation of Kluyveromyces

Plasmid pAB312 was digested with EcoRV (to target integration to the LAC4 region of the K. lactis genome) and was then used to transform K. lactis strain 2UV21 (a ura3 trp1 lac4 [kil°]) to G418 resistance. The plasmids pAB313 and pAB314 were used to transform strain 2UV21 to G418 resistance. Cultures of transformants 2UV21::pAB312, 2UV21::pAB313 and 2UV21::pAB314 were grown and culture supernatants assayed for chymosin activity as above.

A number of these transformants, as well as an untransformed control strain, were grown for 36 hours in 1 ml of medium composed of 1% yeast extract. 2% peptone. 2% glucose, 0.17% yeast nitrogen base, 50 µg/ml tryptophan and 50 µg/'ml uracil. Culture supernatants were then assayed for chymosin activity after acid activation. All of the transformants were found to secrete activatable chymosin. The results are shown in the following Table 8.

**Table 8**

| Secretion of prochymosin in Kluyveromyces | | | |
|---|---|---|---|
| Strain | Host | Plasmid | Chymosin Activity (relative units/ml culture) |
| 2UV21 | 2UV21 | - | <2 |
| KRN303-1 | 2UV21 | pAB312 | 256 |
| KRN304-4 | 2UV21 | pAB313 | 175 |
| KRN305-2 | 2UV21 | pAB314 | 206 |

Each of the transformants was found to secrete a single prochymosin-related species as judged by SDS polyacrylamide gel electrophoresis of trichloroacetic acid-precipitated culture supernatants. The prochymosin-related protein secreted by pAB312 transformants appeared to be of slightly higher molecular weight than those secreted by pAB313 and pAB314 transformants as determined by electrophoretic mobility.

The major species secreted by KRN303-1 and KRN304-4 were purified by preparative SDS polyacrylamide gel electrophoresis and subjected to gas phase amino acid sequence analysis. The N-terminal sequences of these species are given below.

KRN-303-1
1 5 10 15
Glu-Ala-Asp-Ala-Ser-His-His-Met-Ala-Glu-Ile-Thr-Arg-Ile-Pro

KRN304-4
1 5
Ala-Glu-Ile-Thr-Arg-Ile

These results indicate that the prochymosin-related species secreted by KRN303-1 has undergone no processing of the amino-terminal spacer sequence, while the species secreted from KRN304-4 has the authentic mature prochymosin amino terminus.

### Example 8

### Secretion of t-PA by Kluyveromyces lactis using an amyloglucosidase signal sequence

### A. Cloning of tissue-type plasminogen activator cDNA

A cDNA coding for tissue-type plasminogen activator (t-PA) was obtained in a way similar to that described by Pennica et al. (Nature (1983) 301:214). DNA sequence analysis and restriction mapping confirmed the authenicity of the t-PA cDNA. For expression studies the 2.0 kb Bglll fragment (see Pennica et al.), comprising almost the complete coding region for the mature protein and the 3' noncoding region, was used.

### B. Introduction of the G418 resistance marker in pUC19

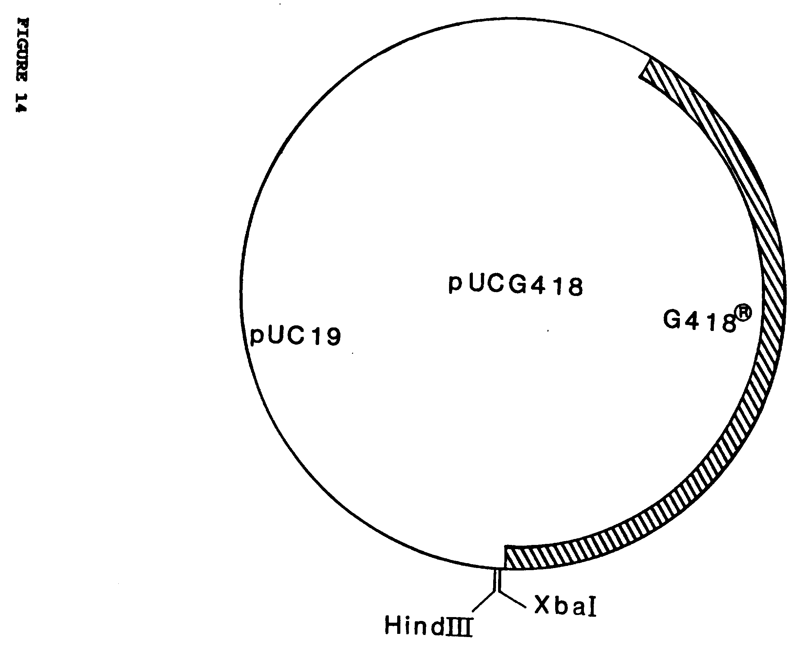

A DNA fragment comprising the Tn5 gene (Reiss et al.. EMBO J. (1984) 3:3317), conferring resistance to G418 under the direction of the alcohol dehydrogenase I (ADHl) promoter from S. cerevisiae similar to that described by Bennetzen and Hall. J. Biol. Chem. (1982) 257:3018, was inserted' into the Smal site of pUC19 (Yanisch-Perron et al., Gene (1985) 33:103). The obtained plasmid, pUCG418, is shown in Figure 14. E. coli containing pUCG418 was deposited at Centraal Bureau voor Schirnmelcultures on December 4, 1987 under CBS 872.87.

### C. Construction of pGBtPA1

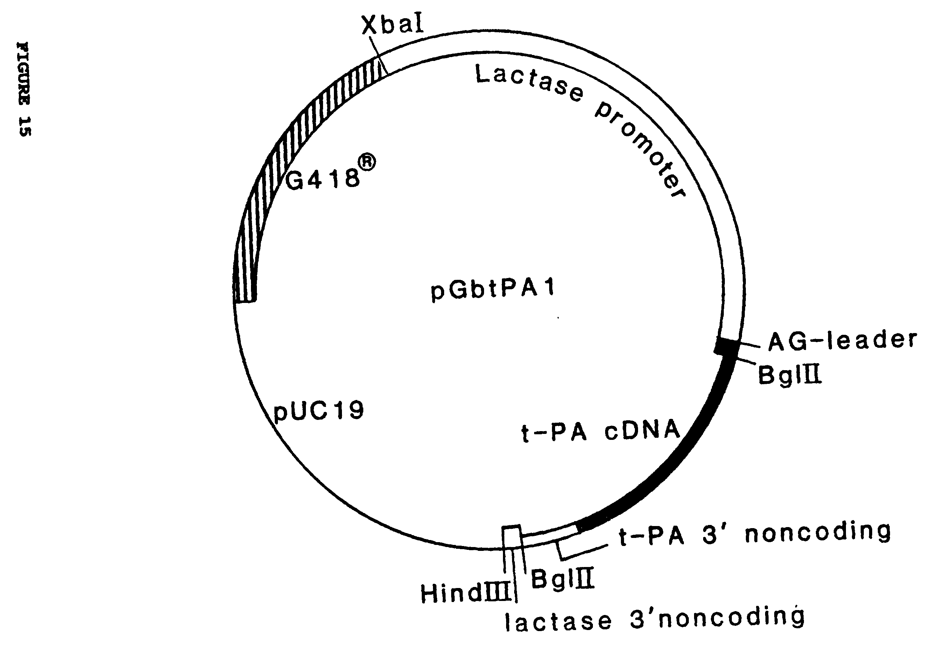

In a few cloning steps pGBtPA1 was constructed (see also Figure 15 and Table 9) containing the following elements:
(1) pUCG418 (see above) cut with Xbal and Hindlll:
(2) the Xbal-Sall fragment from pGB901, containing the lactase promoter:
(3) synthetic DNA coding for the signal sequence of amyloglucosidase from Aspergillus awamori (Innis et al., Science (1985) 228:21). The sequence in front of the startcodon was chosen to remove the Sall site at the end of the lactase promoter fragment and further comprises part of the 5' noncoding region of the lactase gene:
(4) the 2.0 kb Bglll fragment from the t-PA cDNA (see above);
(S) synthetic DNA. comprising part of the 3' noncooing region of the lactase gene.

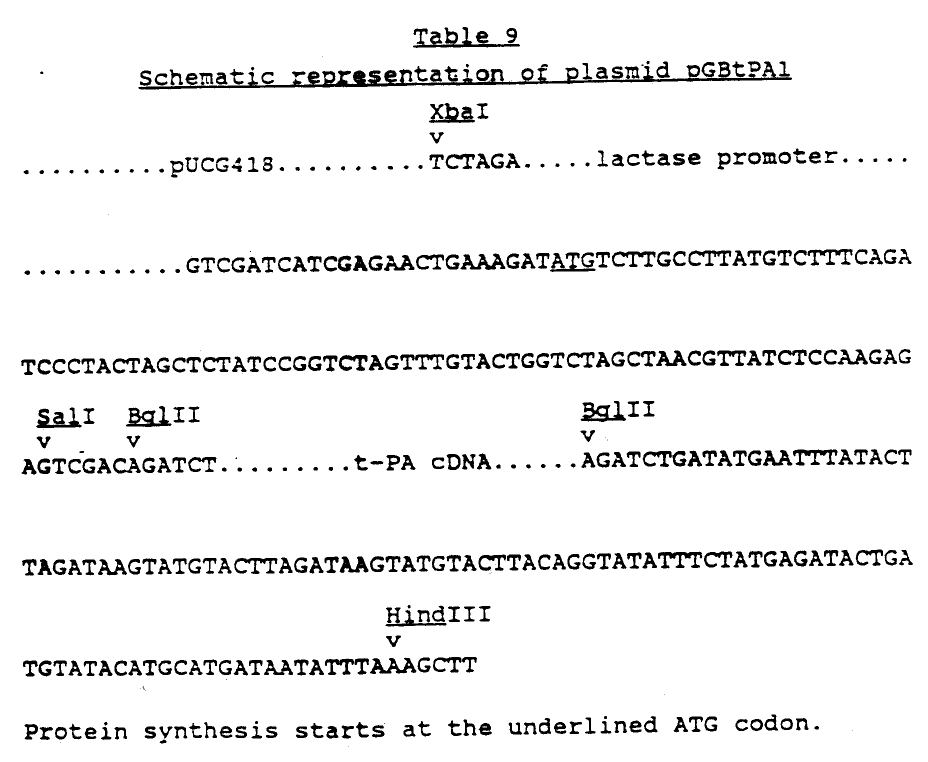

### D. Transformation of. Kluyveromyces lactis and analysis of the transformants.

Transformation of K. lactis strain CBS 2360 with pGBtPA1 was performed as described in Example 2. The transformants and the control strain CBS 2360 were grown in YEPD-medium for about 64 hrs. at 30°C. Calls were separated from the culture medium by centrifugation. The cells were resuspended in a physiological salt solution at an OD₆₁₀ of 300 and disrupted by shaking with glass beads for 3 minutes on a Vortex shaker at maximal speed. Cell debris was removed by centrifugation.

A clotlysis assay according to Wallen et al. (Biochim. Biophys. Acta (1982) 719:318) was performed in microtiter plates. A solution was made, containing 15 mM phosphate buffer (pH 7.3), 0.2 CU/ml plasminogen, 1.5 mg/ml fibrinogen and 0.04% gelatin. Into each well of a microtiter plate 10 µl thrombine (13.9 NIH units/ml), 25 µl sample and 65 µl of the plasminogen/fibrinogen solution were mixed. The reaction was followed by measuring the OD₄₅₀ every 30 min. t-PA from melanorna cells (Kabi Vitrum) was used to provide a calibration curve within every microliter plate.

Table 10 shows the result of a typical analysis of 10 transformants. It is demonstrated that t-PA was found in the culture medium of K. lactis transformed with pGBtPA1.

**Table 10**

| Clotlysis assay of the cultures from CBS 2360 and from CBS 2360 transformed with pGBtPA1 | |
|---|---|
| transformant | t-PA activity in supernatant |
| 1 | 40 µg/l |
| 2 | 6 µg/l |
| 3 | <3 µg/l |
| 4 | <3 µg/l |
| 5 | 25 µg/l |
| 6 | 3 µg/l |
| 7 | 3 µg/l |
| 8 | <3 µg/l |
| 9 | 3 µg/l |
| 10 | <3 µg/l |
| CBS 2360 1°) | <3 µg/l |
| CBS 2360 2°) | <3 µg/l |
| CBS 2360 3°) | <3 µg/l |
| CBS 2360 4°) | <3 µg/l |
| CBS 2360 5°) | <3 µg/l |
| In some of the cell extracts a slight t-PA activity (≦ 3 µg/l) was found. | |

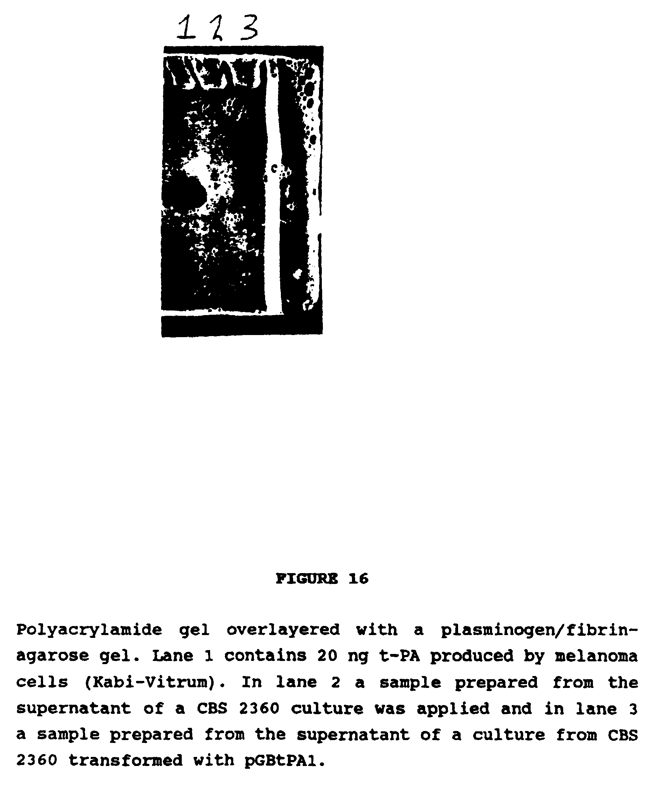

Analysis was also performed on SDS-polyacrylamide gels overlayered with a plasminogen/fibrinagarose gel according to Granelli-Piperno and Reich (J. Exp. Med. (1978) 148:223), 200 µl of the supernatant of a culture of CBS 2360 or CBS 2360 transformed with pGBtPA1 was precipitated with ethanol and resuspended in 20 µl sample buffer (62.5 mM Tris-HCl pH 6.8, 2% sodium dodecylsulphate, 10% glycerol, Bromophanol Blue). The samples were layered on the gels without prior boiling. The results (shown in Figure 16) demonstrate the secretion of human t-PA by K. lactis. Furthermore, it is clear that most of the secreted material is glycosylated.

The secretion of t-PA was also confirmed by using an ELISA with a rnonoclonal antibody against human t-PA (ESP5 purchased from Biopool) and by a chromogenic activity assay (a commercial test from Kabi Vitrum).

### Example 9

### Secretion of t-PA by Kluyveromyces lactis using the signal sequence from human serum albumin

### A. Construction of pGBtPA2

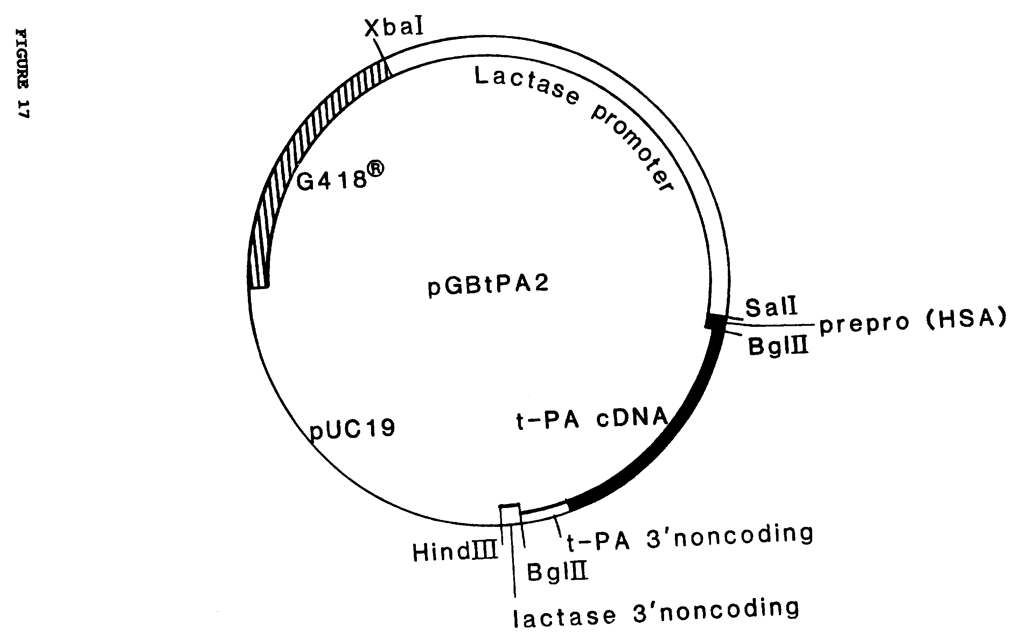

In a few cloning steps pGBtPA2 was constructed (see Figure 17 and Table 11), containing the following elements :
(1) pUCG418. cut with Xbal and Hindlll;
(2) the Xbal-Sall fragment from pGB901, containing the lactase promoter;
(3) synthetic DNA coding for the prepro-region of human serum albumin;
(4) the 2.0 kb Bglll fragment from the t-PA cDNA (see Example 8);
(5) synthetic DNA, comprising part of the 3' noncoding region of the lactase gene.

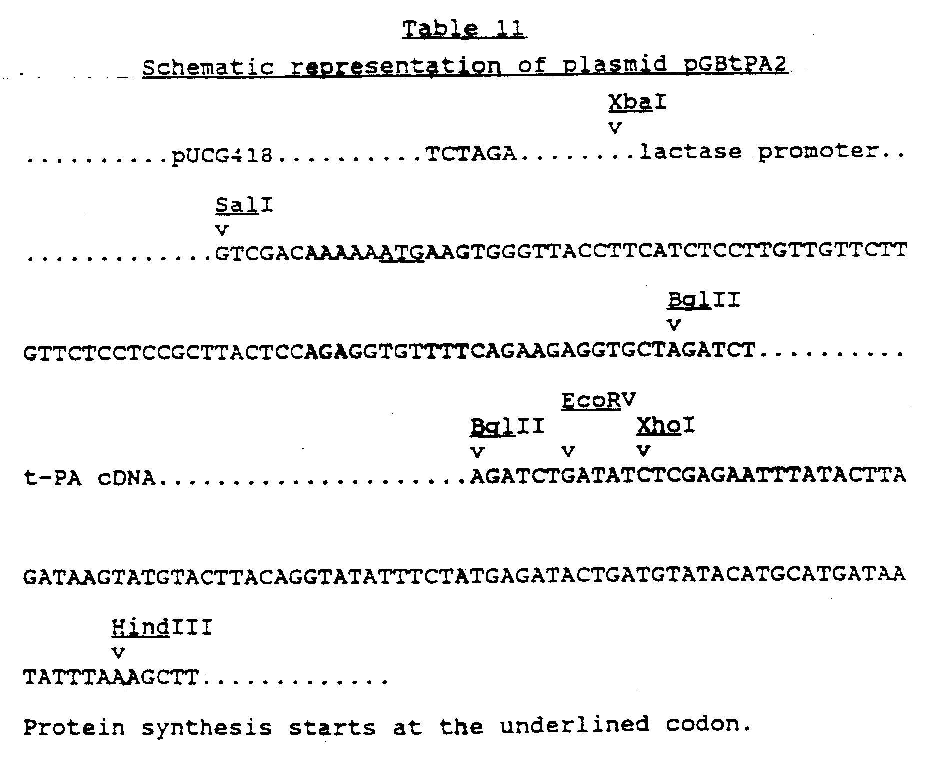

### B. Transformation of K. lactis and analysis of the transformants

Transformation of CBS 2360 with pGBtPA2 was performed as described in Example 8. The transformants and the control strain were grown and treated as described in the previous Example. The results of the clotlysis assay are summarized in Table 12.

**Table 12**

| Clotlysis assay of the cultures of CBS 2360 and CBS 2360 transformed with pGBtPA2 | |
|---|---|
| transformant . | t-PA activity in the supernatant |
| 1 | 25 µg/l |
| 2 | 25 µg/l |
| 3 | <3 µg/l |
| 4 | <3 µg/l |
| 5 | 6 µg/l |
| 6 | 12 µg/l |
| 7 | <3 µg/l |
| 8 | <3 µg/l |
| 9 | <3 µg/l |
| 10 | <3 µg/l |
| CBS 2360 1°) | <3 µg/l |
| CBS 2360 2°) | <3 µg/l |
| CBS 2360 3°) | <3 µg/l |
| CBS 2360 4°) | <3 µg/l |
| CBS 2360 5°) | <3 µg/l |
| In some of the cell extracts a slight t-PA activity (≦3 µg/l) was found. | |

### Example 10

### Secretion of human serum albumin by Kluyveromyces lactis

### A. Synthesis and cloning of the HSA cDNA

cDNA encoding human serum albumin was prepared according to the method of Okayama and Berg (Mol. Cell. Biol. (1982) 2:161) using mRNA prepared from human liver. The cDNA was cloned in a vector derived from pBR322 and transformed to E. coli. Screening of the transformants was performed using a oligodesoxyribonucleotide based on the sequence of the HSA cDNA clone of Lawn et al., Nucleic Acids Res. (1981) 9:6103). The selected cDNA clone was partially sequenced and compared to the sequence of Lawn et al. This revealed that the first 5 nucleotides of the preproHSA coding region were absent, but the BstEll site at nucleotides 9-15 of the coding region was still found. This BstEll site and the Hindlll site in the 3' noncoding region (see Lawn et al., cited above) were used for subcloning in expression vectors.

### B. Construction of pGBHSA1

In a few cloning-steps pGBHSA1 was constructed, containing the following elements:
(1) pUCG418 (see Example 8) cut with Xbal and Hindlll:
(2) the Xbal-Sall fragment from pGB901 (see Example 1);
(3) synthetic DNA (Sall-Hindlll fragment), comprising part of the 3' noncoding region of the lactase gene.

The sequence of this fragment is given in Table 13.

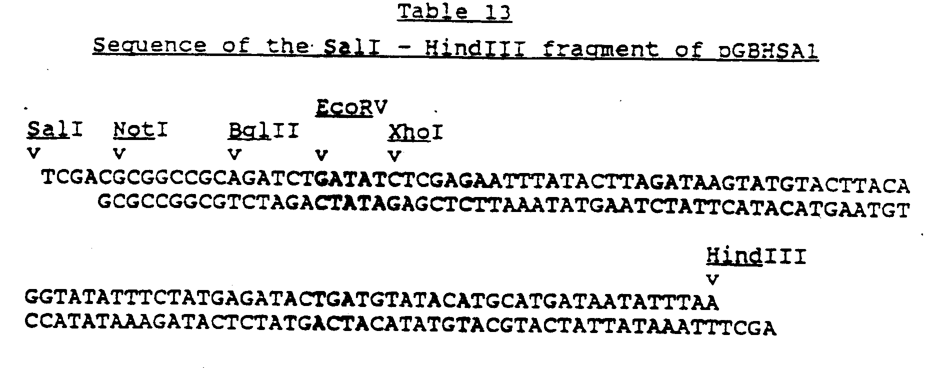

### C. Construction of pGBHSA2

pGBHSA1 was cut with SAll and EcoRV and synthetic DNA was inserted:

SalI                    BstEII          StuI
v                       v               v
 TCGACAAAAATGAAGTGGGTAACCATCGATAGGCCTACTGGGCTCGAGATC
     GTTTTTACTTCACCCATTGGTAGCTATCCGGATGACCCGAGCTCTAG

The underlined ATG-codon indicate the initiation codon in the ultimate expression construct (pGBHSA3, see below).

The resulting plasmid was named pGBHSA2.

### D. Construction of pGBHSA3

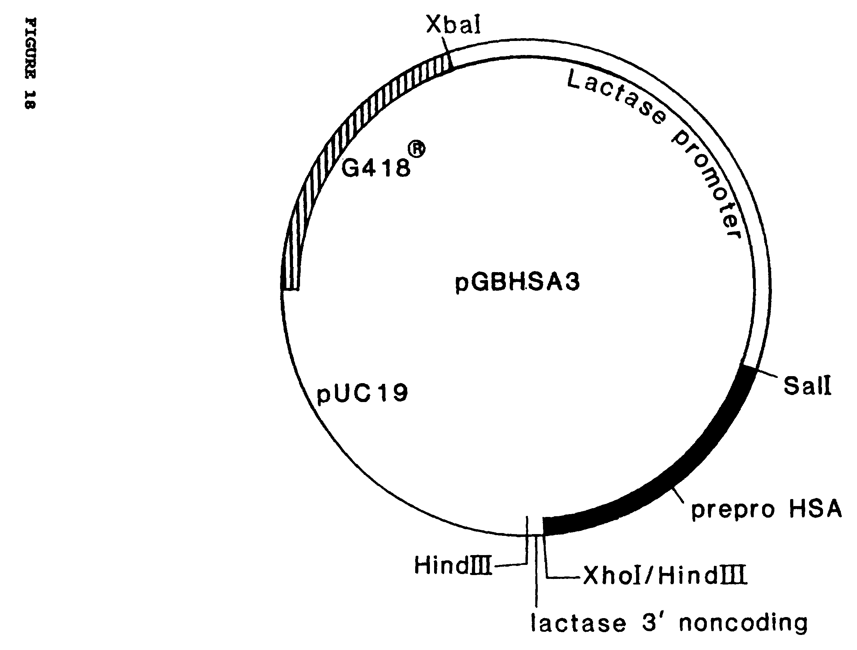

The HSA cDNA-clone was cut with Hindlll and the sticky end was filled in, using Klenow DNA polymerase I. Subsequently the DNA was cut with BstEll and the BstEll-Hindlll (filled in) fragment, containing almost the complete HSA coding region was purified. pGBHSA2 was digested with Xhol, the sticky ends were filled in using Klenow DNA polymerase I and digestion with BstEll was performed. In the resulting vector the HSA encoding fragment (BstEll-Hindlll (filled in)) was inserted. The obtained plasmid. pGBHSA3, is shown in Figure 18.

### E. Transformation of Kluyveromyces lactis and analysis of the transformants

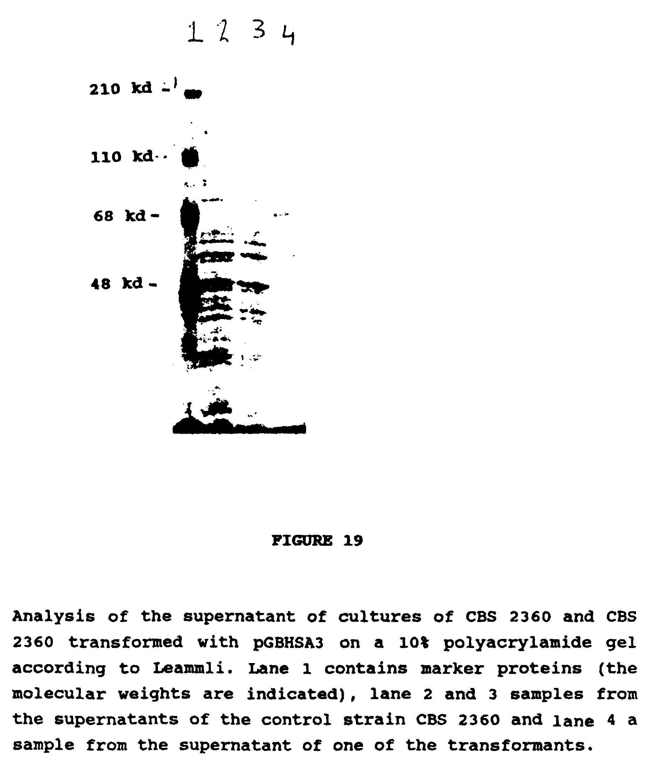

Transformation of K. lactis strain 2360 with pGBHSA3 was performed as described in Example 2. Transformants and the control strain CBS 2360 were grown in YEPD medium for about 64 hrs at 30°C. The cells were separated from the culture medium by centrifugation. Samples of 30 µl were taken from the supernatants and analysed by electrophoresis on a 10% polyacrylamide gel according to Laemmli (Nature 227, 680 (1970)). The results shown in Figure 19 demonstrate that HSA is secreted into the culture medium by K. lactis cells transformed with pGBHSA3. There is also an indication that the secretion of other proteins is reduced in the HSA producing cells.

The above results demonstrate that one can obtain efficient convenient expression of exogenous genes in Kluyveromyces strains. Furthermore, the Kluyveromyces strains appear to be particularly useful for providing highly efficient secretion and processing of a wide variety of proteins, as illustrated by the results with prochymosin. Constructs and vectors are provided which allow for the introduction of an exogenous gene under the regulatory control of efficient promoters in Kluyveromyces and, as desired, joining to signal sequences which provide for translocation of the exogenous gene. particularly secretion. Thus, a fermentaton system is provided for commercial production of a wide variety of exogenous proteins in an active or activatable form.

The following organisms have been deposited with `he American Type Culture Collection on June 30, 1987: 2UV21, ATCC Accession No. 20855; KRN201-6, ATCC Accession No. 20854: HB101 pAB307, ATCC Accession No. 67454; HB101 pAB312, ATCC Accession No. 67455.

Although the foregoing invention has been described in some detail by way of illustration and example for purposes of clarity of understanding, it will be obvious that certain changes and modifications may be practiced within the scope of the appended claims.

## Claims

1. A method for producing a polypeptide of interest in a *Kluyveromyces* host cell, said method comprising:
introducing into said host cell a DNA sequence encoding said polypeptide of interest and
growing said host cell comprising said DNA sequence in a culture medium whereby said polypeptide of interest, or part thereof, is secreted into the culture medium, whereby said polypeptide, or part thereof, is isolated from the culture medium.

2. A method according to Claim 1, whereby said DNA sequence forms part of a DNA construct which is introduced into said host cell and which comprises, in the direction of transcription, a transcriptional initiation regulatory region functional in said host cell; a signal sequence for secretion heterologous to said host cell or to said polypeptide of interest or to said host and to said polypeptide of interest, joined in reading frame to the 5' terminus of said DNA sequence; said DNA sequence encoding said polypeptide of interest; and a transcriptional termination regulatory region functional in said host cell; whereby said polypeptide of interest is secreted by said host cell.

3. A method according to Claim 1 or 2, wherein said polypeptide of interest is an enzyme.

4. A method according to Claim 3, wherein said enzyme is chymosin, or a precursor thereof.

5. A method according to Claim 3, wherein said enzyme is tissue plasminogen activator (t-PA), or mutant forms thereof.

6. A method according to Claim 1 or 2, wherein said polypeptide of interest is human serum albumin (HSA).

7. A method according to any one of Claims 1 to 6, wherein said host cell is an industrial strain of *Kluyveromyce*s.

8. A method according to any one of Claims 1 to 7, wherein said host cell is *K*. *lactis* or *K. fragilis*.

9. A method according to Claim 2, wherein said DNA construct further comprises at least one of a selection marker, a replication system for autonomous replication of said DNA sequence, or a transformation efficiency enhancing sequence.

10. A method according to Claim 9, wherein said replication system is an autonomously replication sequence (ARS).

11. A method according to Claim 10, wherein said autonomously replicating sequence is a *Kluyveromyces* autonomously replicating sequence (KARS).

12. A method according to Claim 11, wherein said selection marker is resistant to G418.

13. Use of *Kluyveromyces* as a host for the transformation and expression of foreign genes and the secretion of the polypeptide encoded by said gene, or secretion of part of said polypeptide, whereby said polypeptide, or part thereof, is isolated from the culture medium.

14. A transformed *Kluyveromyces* host cell comprising an expression cassette which comprises, in the direction of transcription, a transcriptional initiation regulatory region functional in said host cell, a signal sequence for secretion functional in said host cell joined in reading frame with a DNA sequence encoding a polypeptide of interest, and a transcriptional termination regulatory region functional in said host cell, with the proviso that said "a signal sequence for secretion functional in said host cell joined in reading frame with a DNA' sequence encoding a polypeptide of interest"', is not a structural gene coding for methionyl-prochymosin, methionyl-preprochymosin, preprothaumatin, or prethaumatin.

15. A cell according to Claim 14, wherein said signal sequence is heterologous to said host cell or to said polypeptide of interest or to said host cell and to said polypeptide of interest.

16. A cell according to Claim 15, wherein said signal sequence is the α-factor signal sequence from *Saccharomyces cerevisiae*.

17. A cell according to Claim 15, wherein said signal sequence is the amyloglucosidase signal sequence from *Aspergillus awamori*.

18. A cell according to any one of Claims 14 to 17, wherein said polypeptide of interest is chymosin, or a precursor thereof, t-PA or HSA.

19. A cell according to any one of Claims 14 to 18, wherein joined to said expression cassette is at least one of a selection marker, a replication system for autonomous replication of said DNA sequence, or a transformation efficiency enhancing sequence.

20. A cell according to Claim 19, wherein said replication system is an autonomously replicating sequence (ARS).

21. A cell according to Claim 20, wherein said autonomously replicating sequence is a *Kluyveromyces* autonomously replicating sequence (KARS).

22. A cell according to any of Claims 19 to 21, wherein said selection marker is resistant to G418.

23. A DNA construct comprising in the direction of transcription, a *Kluyveromyces* transcriptional initiation regulatory region; a signal sequence for secretion functional in *Kluyveromyces* joined in reading frame with a DNA sequence encoding a polypeptide of interest; and a transcriptional termination regulatory region functional in *Kluyveromyces*, with the proviso that said "a signal sequence for secretion functional in *Kluyveromyces* joined in reading frame with a DNA sequence encoding a polypeptide of interest", is not a structural gene coding for methionyl-prochymosin, methionyl-preprochymosin or preprothaumatin.

24. A DNA construct according to Claim 23, wherein said signal sequence is heterologous to *Kluyveromyces* or to said polypeptide of interest, or to said host cell and said polypeptide of interest.

25. A DNA construct according to Claim 23 or 24, further comprising:
at least one of a selection marker, a replication system for autonomous replication of said DNA construct, or a transformation efficiency enhancing sequence.

26. A DNA construct according to any one of Claims 23 to 25, wherein the DNA fragment encoding the signal sequence and the DNA sequence encoding the polypeptide of interest are fused in reading frame to a gene expressed in *Kluyveromyces*.

27. A DNA construct according to Claim 26, wherein said DNA sequence is fused to second DNA sequence encoding at least four amino acids of the N-terminus of the lactase gene.

28. A plasmid selected from the group consisting of:
pKS105, having a physical map as represented in Fig. 7 herein and comprising an LAC4 promoter/α-factor leader/prochymosin fusion,
pGB901, having a physical map as represented in Fig. 2 herein and comprising a lactase promoter, a gene encoding prochymosin, a lactase terminator and a gene encoding the G418 antibiotic resistance marker,
pGBTPA1, having a physical map as represented in Fig. 15 herein and comprising a gene encoding the G418 antibiotic resistance marker, a fragment from pGB901 containing the lactase promoter, synthetic DNA encoding the signal sequence of amyloglucosidase from *Aspergillus awamori*, a 2.0 kb Bglll fragment from the t-PA cDNA, and synthetic DNA comprising pad of the 3' noncoding region of the lactase gene, and
pGBHSA3, having a physical map as represented in Fig. 18 herein and comprising a gene encoding the G418 antibiotic resistance marker, a fragment from pGB901 containing the lactase promoter, a cDNA fragment encoding preproHSA, and synthetic DNA comprising part of the 3' noncoding region of the lactase gene.

## Patentansprüche

1. Verfahren zur Herstellung eines interessierenden Polypeptids in einer Kluyveromyces-Wirtszelle, wobei das Verfahren umfasst:
Das Einführen in die genannte Wirtszelle einer DNA-Sequenz, die für das genannte interessierende Polypeptid codiert, und
das Kultivieren der genannten Wirtszelle, die die genannte DNA-Sequenz aufweist, in einem Kulturmedium, wodurch das genannte interessierende Polypeptid oder ein Teil davon in das Kulturmedium sekretiert wird, wodurch das genannte Polypeptid oder ein Teil davon aus dem Kulturmedium isoliert wird.

2. Verfahren nach Anspruch 1, bei dem die genannte DNA-Sequenz Bestandteil eines DNA-Konstrukts ist, das in die genannte Wirtszelle eingeführt wird und das in der Transkriptionsrichtung eine Regulationsregion für die Initiation der Transkription, die in der genannten Wirtszelle funktionsfähig ist, eine Signalsequenz für die Sekretion, die in Bezug auf die genannte Wirtszelle oder in Bezug auf das genannte interessierende Polypeptid oder in Bezug auf den genannten Wirt und in Bezug auf das genannte interessterende Polypeptid heterolog ist und im Leseraster mit dem 5'-Terminus der genannten DNA-Sequenz verbunden ist, wobei die genannte DNA-Sequenz für das genannte interessierende Polypeptid codiert, und eine Regulationsregion für die Termination der Transkription, die in der genannten Wirtszelle funktionsfähig ist, aufweist, wodurch das genannte interessierende Polypeptid durch die genannte Wirtszelle sekretiert wird.

3. Verfahren nach Anspruch 1 oder 2, dadurch gekennzeichnet, dass das genannte interessierende Polypeptid ein Enzym ist.

4. Verfahren nach Anspruch 3, dadurch gekennzeichnet, dass das genannte Enzym Chymosin oder ein Vorläufer davon ist.

5. Verfahren nach Anspruch 2, dadurch gekennzeichnet, dass das genannte Enzym Gewebeplasminogenaktivator (t-PA) oder Mutantenformen davon ist.

6. Verfahren nach Anspruch 1 oder 2, dadurch gekennzeichnet, dass das genannte interessierende Polypeptid menschliches Serumalbumin (HSA) ist.

7. Verfahren nach einem der Ansprüche 1 bis 6, dadurch gekennzeichnet, dass die genannte Wirtszelle ein industrieller Stamm von Kluyveromyces ist.

8. Verfahren nach einem der Ansprüche 1 bis 7, dadurch gekennzeichnet, dass die genannte Wirtszelle K. lactis oder K. fragilis ist.

9. Verfahren nach Anspruch 2, dadurch gekennzeichnet, dass das genannte DNA-Konstrukt ferner einen Selektionsmarker und/oder ein Replikationssystem für die autonome Replikation der genannten DNA-Sequenz und/oder eine die Transformationseffizienz steigernde Sequenz aufweist.

10. Verfahren nach Anspruch 9, dadurch gekennzeichnet, dass das genannte Replikationssystem eine autonom replizierende Sequenz (ARS) ist.

11. Verfahren nach Anspruch 10, dadurch gekennzeichnet, dass die genannte autonom replizierende Sequenz eine autonom replizierende Kluyveromyces-Sequenz (KARS) ist.

12. Verfahren nach Anspruch 11, dadurch gekennzeichnet, dass der Selektionsmarker gegen G418 resistent ist.

13. Verwendung von Kluyveromyces als Wirt für die Transformation und Expression von Fremdgenen und die Sekretion des Polypeptids, für das das genannte Gen codiert, oder die Sekretion eines Teils des genannten Polypeptids, wobei das genannte Polypeptid oder ein Teil davon aus dem Kulturmedium isoliert wird.

14. Transformierte Kluyveromyces-Wirtszelle, die eine Expressionskassette aufweist, die in der Transkriptionsrichtung eine Regulationsregion für die Initiation der Transkription, die in der genannten Wirtszelle funktionsfähig ist, eine Signalsequenz für die Sekretion, die in der genannten Wirtszelle funktionsfähig ist und im Leseraster mit einer DNA-Sequenz verbunden ist, die für ein interessierendes Polypeptid codiert, und eine Regulationsregion für die Termination der Transkription, die in der genannten Wirtszelle funktionsfähig ist, aufweist, mit der Maßgabe, daß die genannte "Signalsequenz für die Sekretion, die in der genannten Wirtszelle funktionsfähig ist und im Leseraster mit einer DNA-Sequenz verbunden ist, die für ein interessierendes Polypeptid codiert", kein Strukturgen ist, das für Methionylprochymosin, Methionylpreprochymosin, Preprothaumatin oder Prethaumatin codiert.

15. Zelle nach Anspruch 14, dadurch gekennzeichnet, dass die genannte Signalsequenz heterolog in Bezug auf die genannte Wirtszelle oder in Bezug auf das genannte interessierende Polypeptid oder in Bezug auf die genannte Wirtszelle und in Bezug auf das genannte interessierende Polypeptid ist.

16. Zeile nach Anspruch 15, dadurch gekennzeichnet, dass die genannte Signalsequenz die α-Faktorsignalsequenz aus Saccharomyces cerevisiae ist.

17. Zelle nach Anspruch 15, dadurch gekennzeichnet, dass die genannte Signalsequenz die Amyloglucosidasesignalsequenz aus Aspergillus awamori ist.

18. Zelle nach einem der Ansprüche 14 bis 17, dadurch gekennzeichnet, dass das genannte interessierende Polypeptid Chymosin oder ein Vorläufer davon, t-PA oder HSA ist.

19. Zelle nach einem der Ansorüche 14 bis 18, dadurch gekennzeichnet, dass mit der genannten Expressionskassette mindestens ein Selektionsmarker und/oder mindestens ein Replikationssystem für die autonome Replikation der genannten DNA-Sequenz und/oder eine die Transformationseffizienz steigernde Sequenz verbunden ist.

20. Zelle nach Anspruch 19, dadurch gekennzeichnet, dass das genannte Replikationssystem eine autonom replizierende Sequenz (ARS) ist.

21. Zelle nach Anspruch 20, dadurch gekennzeichnet, dass die genannte autonom replizierende Sequenz eine autonom replizierende Kluyveromyces-Sequenz (KARS) ist.

22. Zelle nach einem der Ansprüche 19 bis 21, dadurch gekennzeichnet, dass der genannte Selektionsmarker gegen G418 resistent ist.

23. DNA-Konstrukt, das in Transkriptionsrichtung eine Regulationsregion für die Initiation der Kluyveromyces-Transkription, eine Signalsequenz für die Sekretion, die in Kluyveromyces funktionsfähig ist und im Leseraster mit einer DNA-Sequenz verbunden ist, die für ein interessierendes Polypeptid codiert, und eine Regulationsregion für die Termination der Transkription, die in Kluyveromyces funktionsfähig ist, aufweist, mit der Maßgabe, daß die genannte "Signalsequenz für die Sekretion, die in Kluyveromyces funktionsfähig ist und im Leseraster mit einer DNA verbunden ist, die für ein interessierendes Polypeptid codiert", kein Strukturgen ist, das für Methionylprochymosin, Methionylpreprochymosin oder Preprothaumatin codiert.

24. DNA-Kontrukt nach Anspruch 23, dadurch gekennzeichnet, dass die genannte Signalsequenz heterolog in Bezug auf Kluyveromyces oder in Bezug auf das genannte interessierende Polypeptid oder in Bezug auf die genannte Wirtszelle und in Bezug auf das genannte interessierende Polypeptid ist.

25. DNA-Konstrukt nach Anspruch 23 oder 24, ferner gekennzeichnet durch mindestens einen Seiektionsmarker und/oder mindestens ein Replikationssystem für die autonome Replikation des genannten DNA-Konstrukts und/oder mindestens eine die Transformationseffizienz steigernde Sequenz.

26. DNA-Konstrukt nach einem der Ansprüche 23 bis 25, dadurch gekennzeichnet, dass das DNA-Fragment, das für die Signalsequenz codiert, und die DNA-Sequenz, die für das interessierende Polypeptid codiert, im Leseraster mit einem Gen fusioniert sind, das in Kluyveromyces exprimiert wird.

27. DNA-Konstrukt nach Anspruch 26, dadurch gekennzeichnet, dass die genannte DNA-Sequenz mit einer zweiten DNA-Sequenz fusioniert ist, die für mindestens vier Aminosäuren des N-Terminus des Lactasegens codiert.

28. Plasmid, das aus der Gruppe ausgewählt ist, die besteht aus:
pKS105 mit einer physikalischen Karte, wie sie in der beiliegenden Figur 7 dargestellt ist, das eine LAC4-Promotor/α-Faktor- Signalsequenz /Prochymosin-Fusion aufweist,
pGB901 mit einer physikalischen Karte, wie sie in der beiliegenden Figur 2 dargestellt ist, das einen Lactasepromotor, ein Gen, das für Prochymosin codiert, einen Lactaseterminator und ein Gen, das für den G418-Antibiotikumresistenzmarker codiert, aufweist,
pGBTPA1 mit einer physikalischen Karte, wie sie in der beiliegenden Figur 15 dargestellt ist, das ein Gen, das für den G418-Antibiotikumresistenzmarker codiert, ein Fragment aus pGB901, das den Lactasepromotor enthält, synthetische DNA, die für die Signalsequenz von Amyloglucosidase aus Aspergillus awamori codiert, ein 2,0 kb-BglII-Fragment aus der t-PA-cDNA und synthetische DNA, die einen Teil der 3'-nichtcodierenden Region des Lactasegens enthält, aufweist, und
pGBHSA3 mit einer physikalischen Karte, wie sie in der beiliegenden Figur 18 dargestellt ist, das ein Gen, das für den G418-Antibiotikumresistenzmarker codiert, ein Fragment aus pGB901, das den Lactasepromotor enthält, ein cDNA-Fragment, das für PreproHSA codiert, und synthetische DNA, die einen Teil der 3'-nichtcodierenden Region des Lactasegens enthält, aufweist.

## Revendications

1. Procédé de production d'un polypeptide intéressant dans une cellule hâte de Kluyveromyces, ledit procédé comprenant:
l'introduction dans ladite cellule hôte d'une séquence d'ADN codant ledit polypeptide intéressant, et
la croissance de ladite cellule hôte comprenant ladite séquence d'ADN dans un milieu de culture de façon que ledit polypeptide intéressant, ou une partie de celui-ci, soit sécrété dans le milieu de culture, de façon que ledit polypeptide, ou une partie de celui-ci, soit isolé à partir du milieu de culture.

2. Procédé selon la revendication 1, dans lequel ladite séquence d'ADN fait partie d'une construction d'ADN qui est introduite dans ladite cellule hôte et qui comprend, dans la direction de transcription, une région régulatrice d'initiation de transcription fonctionnelle dans ladite cellule hôte; une séquence signal pour la sécrétion hétérologue à l'égard de ladite cellule hôte ou dudit polypeptide intéressant ou de ladite cellule hôte et dudit polypeptide intéressant, liée en cadre de lecture à la position 5' terminale de ladite séquence d'ADN; ladite séquence d'ADN codant ledit polypeptide intéressant ; et une région régulatrice de terminaison de transcription fonctionnelle dans ladite cellule hôte; de façon que ledit polypeptide intéressant soit sécrété par ladite cellule hôte.

3. Procédé selon la revendication 1 ou 2, dans lequel ledit polypeptide intéressant est une enzyme.

4. Procédé selon la revendication 3, dans lequel ladite enzyme est la chymosine ou un précurseur de celle-ci.

5. Procédé selon la revendication 3, dans lequel ladite enzyme est l'activateur du plasminogène tissulaire (t-PA) ou des formes mutantes de celui-ci.

6. Procédé selon la revendication 1 ou 2, dans lequel ledit polypeptide intéressant est l'albumine sérique humaine (HSA).

7. Procédé selon l'une quelconque des revendications 1 à 6, dans lequel ladite cellule hôte est une souche industrielle de Kluyveromyces.

8. Procédé selon l'une quelconque des revendications 1 à 7, dans lequel ladite cellule hôte est Kluyveromyces lactis ou Kluyveromyces fragilis.

9. Procédé selon la revendication 2, dans lequel ladite construction d'ADN comprend aussi au moins l'un d'entre un marqueur de sélection, un système de réplication pour la réplication autonome de ladite séquence d'ADN ou une séquence augmentant l'efficacité de la transformation.

10. Procédé selon la revendication 9, dans lequel ledit système de réplication est une séquence à réplication autonome (ARS).

11. Procédé selon la revendication 10, dans lequel la séquence à réplication autonome est une séquence à réplication autonome de Kluyveromyces (KARS).

12. Procédé selon la revendication 11, dans lequel ledit marqueur de sélection est la résistance au G418.

13. Utilisation de Kluyveromyces comme hôte pour la transformation et l'expression de gènes étrangers et la sécrétion de polypeptide codant ledit gène ou la sécrétion d'une partie dudit polypeptide, de façon que ledit polypeptide, ou une partie de celui-ci, soit isolé à partir du milieu de culture.

14. Cellule hôte de Kluyveromyces transformée comprenant une cassette d'expression qui comprend, dans la direction de transcription, une région régulatrice d'initiation de transcription fonctionnelle dans ladite cellule hôte, une séquence signal pour la sécrétion fonctionnelle dans ladite cellule hôte unie en cadre de lecture avec une séquence d'ADN codant un polypeptide intéressant, et une région régulatrice de terminaison de transcription fonctionnelle dans ladite cellule hôte à condition que ladite "séquence signal pour la sécrétion fonctionnelle dans ladite cellule hôte unie en cadre de lecture avec une séquence d'ADN codant un polypeptide intéressant" ne soit pas un gène de structure codant la méthionyl-prochymosine, la méthionyl-préprochymosine, la préprothaumatine ou la préthaumatine.

15. Cellule selon la revendication 14, dans laquelle ladite séquence signal est hétérologue à l'égard de ladite cellule hôte ou dudit polypeptide intéressant ou de ladite cellule hôte et dudit polypeptide intéressant.

16. Cellule selon la revendication 15, dans laquelle la séquence signal est la séquence signal du facteur a de Saccharomyces cerevisiae.

17. Cellule selon la revendication 15, dans laquelle la séquence signal est la séquence signal de l'amyloglucosidase d'Aspergillus awamori.

18. Cellule selon l'une quelconque des revendications 14 à 17, dans laquelle ledit polypeptide intéressant est la chymosine ou un précurseur de celle-ci, le t-PA ou la HSA.

19. Cellule selon l'une quelconque des revendications 14 à 18, dans laquelle est uni à ladite cassette d'expression au moins l'un d'entre un marqueur de sélection, un système de réplication pour la réplication autonome de ladite séquence d'ADN ou une séquence augmentant l'efficacité de la transformation.

20. Cellule selon la revendication 19, dans laquelle ledit système de réplication est une séquence à réplication autonome (ARS).

21. Cellule selon la revendication 20, dans laquelle la séquence à réplication autonome est une séquence à réplication autonome de Kluyveromyces (KARS).

22. Cellule selon l'une quelconque des revendications 19 à 21, dans laquelle ledit marqueur de sélection est la résistance au G418.

23. Construction d'ADN comprenant
dans la direction de transcription, une région régulatrice d'initiation de transcription de Kluyveromyces; une séquence signal pour la sécrétion fonctionnelle dans Kluyveromyces unie en cadre de lecture avec une séquence d'ADN codant un polypeptide intéressant; et une région régulatrice de terminaison de transcription fonctionnelle dans Kluyveromyces, à condition que ladite "séquence signal pour la sécrétion fonctionnelle dans Kluyveromyces unie en cadre de lecture avec une séquence d'ADN codant un polypeptide intéressant" ne soit pas un gène de structure codant la méthionyl-prochymosine , la méthionyl-préprochymosine ou la préprothaumatine.

24. Construction d'ADN selon la revendication 23, dans laquelle ladite séquence signal est hétérologue à l'égard de Kluyveromyces ou dudit polypeptide intéressant ou de ladite cellule hôte et dudit polypeptide intéressant.

25. Construction d'ADN selon les revendications 23 ou 24, comprenant aussi :
au moins l'un d'entre un marqueur de sélection, un système de réplication pour la réplication autonome de ladite construction d'ADN ou une séquence augmentant l'efficacité de la transformation.

26. Construction d'ADN selon une quelconque des revendications 23 à 25, dans laquelle le fragment d'ADN codant la séquence signal et la séquence d'ADN codant le polypeptide intéressant sont fusionnés en cadre de lecture à un gène exprimé dans Kluyveromyces.

27. Construction d'ADN selon la revendication 26, dans laquelle ladite séquence d'ADN est fusionnée avec une deuxième séquence ADN codant au moins quatre acides aminés en position N-terminale du gène lactase.

28. Plasmide choisi parmi le groupe constitué par:
pKS105, ayant une carte physique telle que représentée dans la Fig. 7 en annexe et comprenant une fusion promoteur LAC4/ séquence de tête du facteur α/prochymosine,
pGB901, ayant une carte physique telle que représentée dans la Fig. 2 en annexe et comprenant un promoteur lactase, un gène codant la prochymosine, un terminateur lactase et un gène codant le marqueur de résistance à l'antibiotique G418,
pGBTPA1, ayant une carte physique telle que représentée dans la Fig. 15 en annexe et comprenant un gène codant le marqueur de résistance à l'antibiotique G418, un fragment de pGB901 contenant le promoteur lactase, l'ADN synthétique codant la séquence signal de lamyloglucosidase d'Aspergillus awamori, un fragment BglII de 2,0 kb de l'ADNc de t-PA et un ADN synthétique comprenant une partie de la région 3' non codante du gène lactase, et
pGBHSA3, ayant une carte physique telle que représentée dans la Fig. 18 en annexe et comprenant un gène codant le marqueur de résistance à l'antibiotique G418, un fragment de pGB901 contenant le promoteur lactase, un fragment d'ADNc codant la preproHSA et un ADN synthétique comprenant une partie de la région 3' non codante du gène lactase.
